# EUROPÄISCHE PATENTSCHRIFT

(11) **EP 1 658 047 B1**
(45) Veröffentlichungstag und Bekanntmachung des Hinweises auf die Patenterteilung: **12.03.2008**
(21) Anmeldenummer: 04764317.6
(22) Anmeldetag: 20.08.2004
(51) Int. Cl.: A61K 9/00, A61K 47/00

(54) **SPRÜHGETROCKNETE AMORPHE PULVER MIT GERINGER RESTFEUCHTE UND GUTER LAGERSTABILITÄT**
SPRAY-DRIED AMORPHOUS POWDER WITH A LOW RESIDUAL MOISTURE AND EXCELLENT STORAGE STABILITY
POUDRES AMORPHES SECHEES PAR ATOMISATION PRESENTANT UNE FAIBLE HUMIDITE RESIDUELLE ET UNE BONNE STABILITE AU STOCKAGE

(30) Priorität: 22.08.2003 DE 10339197
(43) Veröffentlichungstag der Anmeldung: 24.05.2006
(73) Patentinhaber: Boehringer Ingelheim Pharma GmbH & Co. KG, 55216 Ingelheim (DE)
(72) Erfinder: BECHTOLD-PETERS, Karoline, 88400 Biberach-Rissegg (DE); FRIESS, Wolfgang, 82393 Iffeldorf (DE); SCHUELE, Stefanie, 80798 München (DE); BASSARAB, Stefan, 88400 Biberach (DE); GARIDEL, Patrick, 22846 Norderstedt (DE); SCHULTZ-FADEMRECHT, Torsten, 88437 Maselheim, OT Äpfingen (DE)
(86) Internationale Anmeldenummer: PCT/EP2004/009333
(87) Internationale Veröffentlichungsnummer: WO 2005/020953

(56) Entgegenhaltungen:
- EP-A- 0 656 204
- WO-A-00/10541
- WO-A-01/93829
- WO-A-96/09814
- WO-A-96/32149
- WO-A-98/16205
- WO-A-98/31346
- WO-A-02/101412
- WO-A-03/043574
- WO-A-03/087335
- US-A1- 2003 133 879
- US-B1- 6 284 282

## Beschreibung

### Gebiet der Erfindung

Die Erfindung betrifft sprühgetrocknete, amorphe und lagerstabile protein-/peptidhaltige Pulver sowie Verfahren zu deren Herstellung. Die Erfindung betrifft im Besonderen die Herstellung von Mannitol enthaltenden protein-/peptidhaltigen Pulvern mittels Sprühtrocknung und geeigneter Nachtrocknung, wobei das Pulver eine Restfeuchte von weniger als 1.2% (w/w) aufweist und als einen oder mehrere zusätzliche Hilfsstoffe Tripeptide der allgemeinen Formel Ile-X-X, X-Ile-X, X-X-Ile enthält.

### Hintergrund

Durch die fortschreitende Entwicklung der Biotechnologie hat die Anzahl an pharmazeutischen Zubereitungen mit peptid- oder proteinhaltigen Wirkstoffen stark zugenommen. Peptide/Proteine unterliegen häufig physikalischen und chemischen Instabilitäten, sobald sie länger in wässrigen Lösungen vorliegen (Cleland et al. 1993, Crit. Rev. Ther. Drug Carrier Syst. 10(4), 307-377). Zu den chemischen Instabilitäten gehören beispielsweise Deamidierung, Hydrolyse, Racemisierung, Oxidation, β-Eliminierung und Disulfidbrückentausch. Als physikalische Instabilitäten kennt man die Denaturierung mit anschließender Aggregation oder Adsorption sowie die direkte Aggregation mit anschließender Präzipitation. Diese Mechanismen, die sich unmittelbar auf die Struktur des Peptids/Proteins auswirken, führen zumeist zu einer Verringerung ihrer Bioaktivität und somit ihrer therapeutischen Wirkung.

Peptide/Proteine lassen sich durch Trocknungsverfahren stabilisieren. Es sind verschiedene Verfahren zur Herstellung von trockenen peptid-/proteinhaltigen pharmazeutischen Zusammensetzungen bekannt. Weit verbreitet sind beispielsweise Gefriertrocknungsverfahren (Franks, et al., 1990, Cryo Lett., 11, 93-11; Pikal et al., 1990, Biopharm. 3(9), 26-30; Hora et al., 1992, Pharm. Res. 8(3), 285-291; Franks et al., 1992, Jap. J. Freezing Drying 38, 15-16; WO 02/101412). Entsprechende Verfahren sind jedoch nachteilig, da viele Proteine beim Einfrieren geschädigt werden oder aber physiologisch inakzeptable Hilfsmittel zu Stabilisierung benötigt werden. Alternativen hierzu stellen Vakuumtrocknung oder Sprühtrocknungsverfahren dar (Franks et al., 1993, in van den Teel et al., (Eds.) Stability and Stabilisation of Enzymes, Elsevier Sci. Publ., 45-54; Roser et al., 1991, Biopharm. 4(9), 47-53; Carpenter et al., 1988; Cryobiol. 25, 459-470).

Ein mittlerweile bevorzugtes Verfahren zur chemischen und physikalischen Stabilisierung von Peptiden/Proteinen und damit zur Herstellung von pulverförmigen peptid-/proteinhaltigen pharmazeutischen Zubereitungen stellt die Sprühtrocknung dar (vgl., Maa et al., 1998, Pharmaceutical Research, 15(5), 768-775). Besonders im Bereich der pulmonalen Therapie ist die Sprühtrocknung ein geeignetes Verfahren um peptid-/proteinhaltige Pulver zur Behandlung verschiedener Erkrankungen herzustellen (US 5,626,874; US 5,972,388; Broadhead et al., 1994, J. Pharm Pharmacol., 46(6), 458-467). Die inhalative Applikation von Peptiden/Proteinen stellt auch bei systemischen Erkrankungen eine Alternative zu traditionellen Applikationsformen, wie beispielsweise der parenteralen Applikation, dar, da inhalativ applizierte Arzneistoffe neben einer lokalen auch eine systemische Wirkung entfalten können (WO 99/07340). Voraussetzung hierbei ist, dass die mittlere Teilchengröße im Bereich von 1-10 µm liegt, so dass die,Partikel in tiefere Lungenabschnitte und somit in den Blutkreislauf gelangen können. Die DE-A-179 22 07 beschreibt beispielhaft die Herstellung von Sprühtrocknungspartikeln, die eine ausreichende Dispergierbarkeit bei der medizinischen Anwendung (Inhalation) aufweisen. Mittlerweile sind eine Vielzahl an Verfahren zur Herstellung entsprechender inhalierbarer Pulver im Stand der Technik bekannt (WO 95/31479; WO 96/09814; WO 96/32096; WO 96/32149; WO 97/41833; WO 97/44013; WO 98/16205; WO 98/31346; WO 99/66903; WO 00/10541; WO 01/13893; Maa et al., 1998, *supra*; Vidgrén et al., 1987, Int. J. Pharmaceutics, 35, 139-144; Niven et al., 1994, Pharmaceutical Research, 11(8), 1101-1109).

Bei der Herstellung von inhalierbaren Pulvern mittels Sprühtrocknung kann es zur Entfaltung, Aggregation und/oder Inaktivierung des Peptids/Proteins kommen (Broadhead et al., 1994, *supra*). Entsprechende Effekte treten sowohl bei dem Sprühvorgang als auch bei der anschließenden Lagerung der Pulver auf. Ursachen hierfür sind beispielsweise auftretende Scherkräfte, hohe Sprühtemperaturen, Oberflächeneffekte, Flüssig-Fest-Interaktionen und der gleichen. Weitere Probleme können darin bestehen, dass die resultierenden trockenen Pulver zu große mittlere Teilchengrößen, schlechte Fließeigenschaft oder eine schlechte Dispergierbarkeit aufweisen. Aus diesem Grund werden Peptide/Proteine während der Sprühtrocknung mit einem oder mehreren Hilfsstoffen (oder auch Matrixbildnern) versprüht. Die Auswahl der Hilfsstoffe hängt unter anderem von ihrer Stabilisierungsfähigkeit ab. Darüber hinaus spielen auch Faktoren wie die pharmazeutische Akzeptanz des Hilfsstoffs sowie dessen Einfluss auf die Teilchenbildung, insbesondere auf die mittlere Teilchengröße, den mittleren aerodynamischen Teilchendurchmesser (MMAD), den Anteil der *Fine Particle Fraction* (FFP), die Dispergierbarkeit und die Fließeigenschaft eine entscheidende Rolle.

Als Hilfsstoffe haben sich unter anderem Zucker und deren Alkohole wie beispielsweise Trehalose, Lactose, Saccharose oder Mannitol, sowie verschiedene Polymere als geeignet erwiesen (Maa et al., 1997, Pharm. Development and Technology, 2(3), 213-223; Maa et al., 1998, supra; Dissertation Adler, 1998, Universität Erlangen; Costantino, et al., 1998, J. of Pharm. Sciences, 87(11), 1406-1411).

Costantino et al., beschreiben, dass Mannitol bei einem Verhältnis 20 zu 80% (w Mannitol / w Protein) eine maximal stabilisierende Wirkung auf Proteine besitzt. Obwohl höhere Anteile an Mannitol eine verbesserte Fließeigenschaft und Proteinstabilisierung erwarten lassen, nimmt die tatsächliche Tendenz zur Proteinstabilisierung mit zunehmendem Mannitol-Anteil ab, da Mannitol zur Auskristallisation neigt. Bei Formulierungen mit 10-30% (w/w) Mannitol nimmt der kristalline Anteil während der Lagerung auf über 10% des Gesamtproteinanteils zu.

Von Maa et al., 1998 (*supra*) ist die Herstellung mannitolhaltiger Antikörperformulierungen zur Inhalation bekannt, die mittels Sprühtrocknung und Nachtrocknung hergestellt werden. Die Restfeuchte für die Formulierung mit 80/20% (w/w) anti-IgE Antikörper/Mannitol lag nach Nachtrocknung bei 2,4% (w/w) (nach der Kart-Fischer-Bestimmung). Der Anteil an Aggregaten betrug nach einer Langzeitlagerung von 15 Wochen bei 30°C bzw. 40°C bis zu 18% am Gesamtproteinanteil. Die dargestellte Pulverperformance ist somit wenig geeignet für die Applikation von pharmazeutischen Wirkstoffen.

Die Eigenschaft von Polyolen, insbesondere von Mannitol, pharmazeutische Wirkstoffe wie Peptide oder Proteine hinreichend zu stabilisieren und Pulver mit einer guten inhalierbaren Performance zu bilden, scheint durch das hohe kristalline Potential der Polyole stark eingeschränkt zu sein.

Eine Aufgabe der vorliegenden Erfindung bestand darin, die stabilisierende Wirkung von Polyolen durch Reduktion der Kristallisation für pharmazeutische Anwendungen, insbesondere für die Formulierung von peptid-/proteinhaltigen sprühgetrockneten Pulvern nutzbar zu machen.

Eine weitere Aufgabe der Erfindung bestand darin, die stabilisierende Wirkung von Polyolen für die Formulierung.von sprühgetrockneten Pulvern nutzbar zu machen, die komplexe Proteine wie beispielsweise Antikörper als pharmazeutischen Wirkstoff enthalten.

Eine grundsätzliche Aufgabe der vorliegenden Erfindung bestand darin, sprühgetrocknete Pulver zur Verfügung zustellen, die sich durch eine gute Langzeitstabilität und Inhalierbarkeit auszeichnen. Entscheidend hierbei ist eine ausgewogen Balance zwischen beiden Kriterien.

Eine weitere Aufgabe der vorliegenden Erfindung bestand darin pharmazeutische Zubereitungen für die inhalative Applikation, sei es in Form eines trockenen Pulvers, eines treibgashaltigen Dosieraerosols oder einer treibgasfreien Inhalationslösung.

Die der Erfindung zugrunde liegenden Aufgaben werden durch die nachfolgenden Ausführungen sowie durch die in den Patentansprüchen dargestellten Gegenstände/Verfahren gelöst.

### Zusammenfassung der Erfindung

Die vorliegende Erfindung betrifft sprühgetrocknete amorphe Pulver, die einen pharmazeutischen Wirkstoff und einen Matrixbildner enthalten. Die Pulver haben einen Feuchtigkeitsgehalt der unter 1.2% (w/w), vorzugsweise unter 1% (w/w) liegt, und enthalten als einen oder mehrere zusätzliche Hilfsstoffe Tripeptide der allgemeinen Formel Ile-X-X, X-Ile-X, X-X-Ile, wobei X eine der folgenden Aminosäuren sein kann: Alanin, Glycin, Arginin, Histidin, Glutaminsäure, Glutamin, Asparagin, Asparaginsäure, Cystein, Leucin, Lysin, Isoleucin (Ile), Valin, Tryptophan, Methionin, Phenylalanin, Prolin, Serin, Threolin, Tyrosin, L-Aspartyl-L-phenylalanin methylester (= Aspartam), Trimethylammonio-acetat und wobei Tle für Isoleucin steht.

In einer bevorzugten Ausführungsform besteht das Pulver überwiegend aus feinteiligen inhalierbaren Partikeln mit einer mittleren aerodynamischen Teilchengröße (mass median aerodynamic diameter = MMAD) von ≤10 µm, vorzugsweise von 0,5-7,5µm, weiter bevorzugt von 1-5µm. Die Matrixbildner können hierbei Zucker, Polyole, Polymere oder eine Kombination aus diesen sein. Die Zucker sind vorzugsweise Mono-, Di-, Oligo- oder Polysaccharide oder eine Kombination hieraus.

In einer besonders bevorzugten Ausführungsform enthalten die Pulver Polyole als Matrixbildner, beispielsweise Mannitol. Der Anteil des Mannitols in den Pulvern beträgt nach einer weiteren Ausführungsform der Erfindung zwischen 20-60% (w/w), vorzugsweise zwischen 20-40% (w/w) der Trockenmasse des Pulvers. Nach einer weiter bevorzugten Ausführungsform der Erfindung beträgt der Polyolanteil, insbesondere der Mannitolanteil, mehr als 20% (w/w), vorzugsweise zwischen 30-60% (w/w), besonders bevorzugt zwischen 30-50% (w/w), noch mehr bevorzugt zwischen 30-40% (w/w).

Die erfindungsgemäßen sprühgetrockneten Pulver können zusätzlich eine oder mehrere oberflächenaktive Substanz(en) und/oder ein Salz(e) enthalten. Ferner können die sprühgetrockneten Pulver weitere Hilfsstoffe, wie beispielsweise Aminosäuren, Peptide, Proteine oder auch Zucker enthalten.

Vorzugsweise handelt es sich bei dem weiteren Hilfsstoff um ein Tri-Peptid mit zumindest einem Isoleucinrest, vorzugsweise um Tri-Isoleucin. Nach einer speziellen Ausführungsform betrifft die vorliegende Erfindung sprühgetrocknete Pulver die in Bezug auf ihre Trockenmasse einen Anteil von **(a)** ungefähr 20 bis 60% (w/w) Matrixbildner, vorzugsweise ein Polyol, wie zum Beispiel Mannitol, **(b)** ungefähr 1 bis 19,99% (w/w) Aminosäuren, vorzugsweise Isoleucin und **(c)** ungefähr 0,01 bis 79% (w/w) eines pharmazeutischen Wirkstoffs, vorzugsweise eines Peptids/Proteins, beispielsweise eines Antikörpers, enthalten. Nach einer weiteren alternativen Ausführungsform betrifft die vorliegende Erfindung sprühgetrocknete Pulver, die in Bezug auf ihre Trockenmasse **(a)** ungefähr 20 bis 60% (w/w) Matrixbildner, vorzugsweise ein Polyol, wie zum Beispiel Mannitol, **(b)** ungefähr 1 bis 19,99% (w/w) eines Isoleucin-haltigen Tri-Peptids, vorzugsweise Tri-Isoleucin und **(c)** ungefähr 0,01 bis 79% (w/w) eines pharmazeutischen Wirkstoffs, vorzugsweise eines Peptids/Proteins, beispielsweise eines Antikörpers, enthalten. Die entsprechenden Pulver, insbesondere nach Beimischung von Isoleucin, oder Isoleucin-haltigen Tri-Peptiden zeigen sehr gute Fließeigenschaften und zeichnen sich durch einen sehr hohen Anteil an inhalierbaren Teilchen aus. Ferner verfügen die entsprechenden Pulver über eine sehr gute Prozess- und Lagerstabilität.

Bei dem pharmazeutischen Wirkstoff handelt es vorzugsweise um ein biologisches Makromolekül, welches ein Polypeptid oder ein Protein sein kann. Die erfindungsgemäßen Pulver enthalten nach einer weiter bevorzugten Ausführungsform Wachstumsfakoren, Enzyme oder Antikörper. Besonders erfindungsgemäß sind inhalierbare sprühgetrocknete Pulver enthaltend **(a)** einen Mannitolanteil von ≥ 20% (w/w), **(b)** Antikörper als pharmazeutischen Wirkstoff, vorzugsweise in einer Konzentration von 0,1-80% (w/w), u n d (c) einer Restfeuchte von ≤ 1,2% (w/w), vorzugsweise von ≤ 1,0% (w/w).

Nach einer weiteren erfindungsgemäßen Ausführungsform beträgt der Anteil an aggregierten Wirkstoff in den erfindungsgemäßen Pulvern in Bezug auf den Gesamtwirkstoffanteil weniger als 3,5%, vorzugsweise weniger als 3%.

Die erfindungsgemäßen Pulver eignen sich zur Formulierung von pharmazeutischen Zusammensetzungen, so dass die vorliegenden Erfindung auch entsprechende pharmazeutischen Zusammensetzungen umfasst, vorwiegend für inhalative Applikationen, die eines der hier beschriebenen erfindungsgemäßen Pulver enthalten. In diesem Zusammenhang besonders bevorzugt sind pharmazeutische Zusammensetzungen, die die erfindungsgemäßen Pulver als treibgashaltige Dosieraerosole oder als treibgasfreie Inhalationslösungen enthalten.

Die Erfindung stellt ferner ein Verfahren, bestehend aus Sprühtrocknung und Vakuumtrocknung, zur Herstellung von Proteinzubereitungen in Gegenwart zumindest eines Hilfsstoffs bereit. Die Temperatur für den Sprühtrocknungsprozess liegt unterhalb von 135/70°C (Einström-/Ausströmtemperatur), vorzugsweise unterhalb von 105/60°C. Das resultierende Pulver wird durch Vakuumtrocknung bei einer Temperatur von 25-60°C, vorzugsweise von 30-60°C nachgetrocknet. Das Vakuum der Trocknung wird zwischen 0,05 und 1 mbar, vorzugsweise zwischen 0,05 und 0,5 mbar, besonders bevorzugt zwischen 0,05 und 0,2 mbar eingestellt.

Die vorliegende Erfindung stellt sprühgetrocknete amorphe Pulver mit verbesserten Eigenschaften in Bezug auf Restfeuchte und den davon abhängigen Eigenschaften wie Fließeigenschaft, Dispergierbarkeit, Lager- und Prozessstabilität zur Verfügung. Die vorliegende Erfindung löst somit Probleme, die sich bei der bisherigen Formulierungsentwicklung, insbesondere bei der Verwendung von mannitolhaltigen Sprütrocknungspulvern ergeben haben, da deren Restfeuchten einen negativen Einfluss auf die physikalische und chemische Stabilität der Pulver hatten. Die Aktivität der Peptide/Proteine wird während der Lagerung und während des Prozesses in den Formulierungen erhalten.

WO 03/087335 offenbart Verfahren zur Herstellung von sprühgetrockneten Pulvern, die einen Nachtrocknungsprozess umfassen. Die beschriebenen Pulver enthalten Matrixbildner und mögliche weitere Hilfsstoffe wie Aminosäuren, aber nicht Peptide, insbesondere keine Tripeptide.

WO 03/043574 beschreibt pharmazeutische Zusammensetzungen, die getrocknet werden und danach einen bestimmten Partikeldurchmesser besitzen. Die beschriebenen Zusammensetzungen sind nicht amorph.

WO 02/101412 beschreibt eine Methode zur Herstellung eines Pulvers, wobei die hergestellten Pulver nicht amorph sind bzw. keine Restfeuchten unter 1.2% (w/w) offenbart sind.

WO 01/93829 beschreibt ein sprühgetrocknetes Pulver, welches aber weder amorph ist, noch geringe Restfeuchte unter 1.2% (w/w) besitzt.

US 6,284,282 beschreibt eine Methode zur Herstellung sprühgetrockneter Pulver, welche eine Nachtrocknung beinhaltet. Bei der beschriebenen Methode ist eine Sprüh-Gefriertrocknung umfasst sowie die Immersion in eine kalte Flüssigkeit.

US 2003/133879 beschreibt Mikropartikel einer bestimmten Größe, die zur inhalativen Applikation geeignet sind. Die beschriebenen Mikropartikel enthalten keine Peptide als Hilfsstoffe.

WO 00/10541 beschreibt eine Methode zur Herstellung sprüh getrockneter Partikel. Die beschriebenen Partikel sind nicht amorph.

WO 98/31346 beschreibt Partikelzusammensetzungen einer bestimmten Größe, die zur inhalativen Applikation geeignet sind. Die beschriebenen Zusammensetzungen enthalten nur Fettsäuren, Phospholipide, Blockcopolymere, Phosphoglyceride und L-alpha-Phosphatidylcholin Dipalmitoyl als Hilfsstoffe.

WO 98/16205 beschreibt eine pulverförmige amorphe Zusammensetzung mit einem Matrixbildner. Die beschriebenen Zusammensetzungen enthalten lediglich Polypeptide und Aminosäuren als Hilfsstoffe, aber keine Isoleucin-haltigen Tripeptide.

EP 0656204 beschreibt Tabletten mit kontrollierter Wirkstoff-Freisetzung. Amorphe sprühgetrocknete Pulver mit Restfeuchte unter 1.2%(w/w) enthaltend Matrixbildner wie Mannitol bzw. Hilfsstoffe wie Isoleucinhaltige Peptide sind nicht beschrieben. Zudem scheinen die beschriebenen Tabletten nicht geeignet für die inhalative Applikationsroute, sondern sie erscheinen vielmehr für die gastro-intestinale Applikationsroute konzipiert zu sein.

WO 96/32149 beschreibt Pulverzusammensetzungen mit einer bestimmten Partikel-Größenverteilung und Restfeuchten unter 3% (w/w). Als mögliche Hiltsstoffe werden lediglich Aminosäuren und Polypeptide aufgeführt.

WO 96/09814 beschreibt Mikropartikel einer bestimmten Größe, die jedoch weder amorph sind noch eine geringe Restfeuchte von kleiner 1.2% (w/w) besitzen.

### Beschreibung der Abbildungen

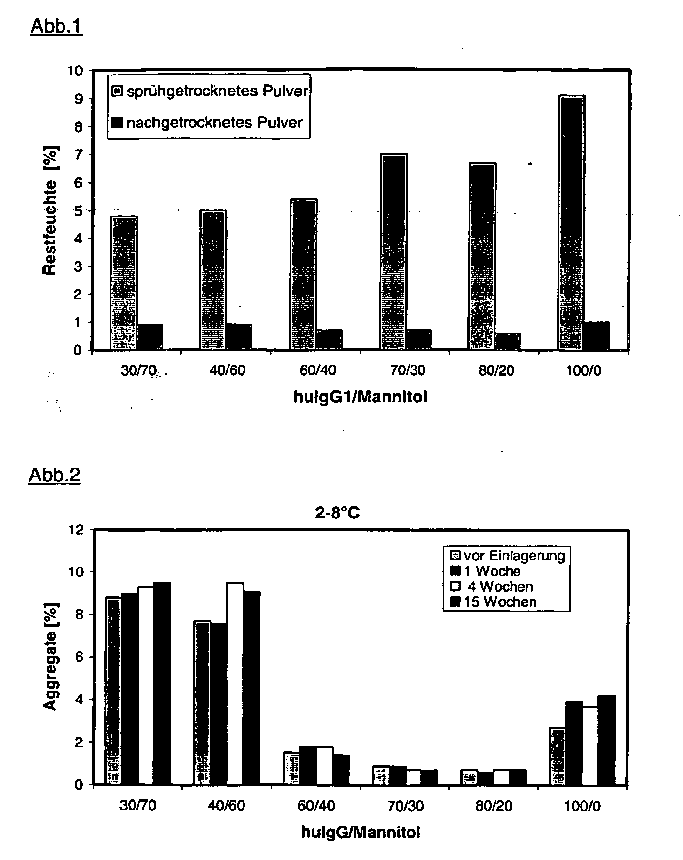

**Abbildung 1** beschreibt den Restwassergehalt (=Restfeuchte) direkt nach der Sprühtrocknung bei einer Raumtemperatur von 22,5°C und 50%rF (= relative Feuchte) und nach der Nachtrocknung unter Vakuum (24 h, 32°C, 0,1 mbar). Man sieht, dass der Restwassergehalt bei allen Formulierungen unter 1 % sinkt. Ohne Nachtrocknung liegt der Restwassergehalt bei 4 bis 9,5%.

**Abbildung 2** zeigt die Aggregatbildung nach Sprühtrocknung und anschließender Vakuumtrocknung vor der Einlagerung, nach 1, 4 und 15 Wochen Lagerung bei 2-8°C unter Stickstoffverschluss und in Aluminiumbeutel eingeschweißt.

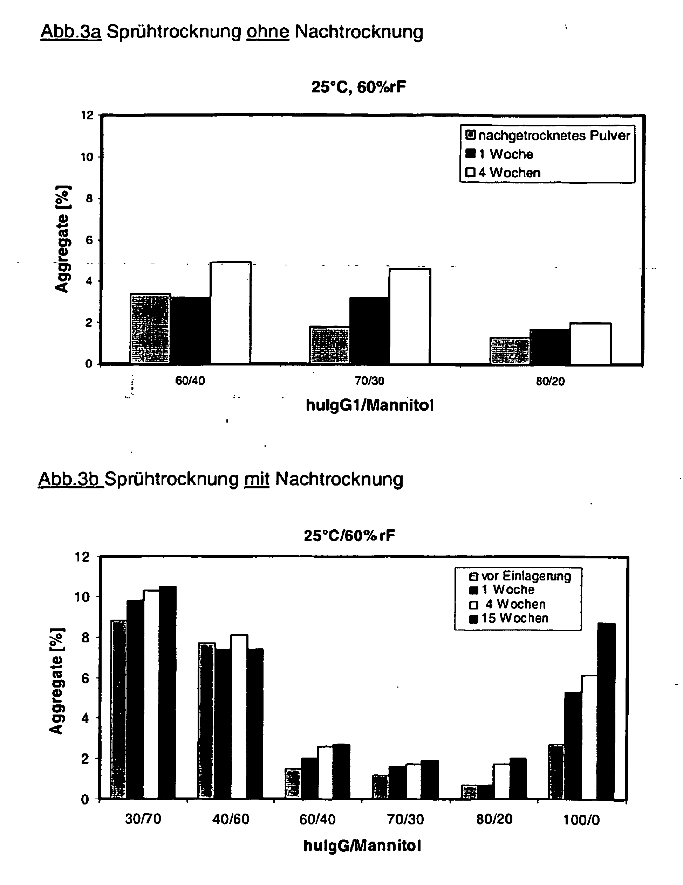

**Abbildung 3** zeigt die Aggregatbildung nach Sprühtrocknung mit und ohne anschließende Vakuumtrocknung jeweils vor Einlagerung und nach 1, 4 und 15 Wochen Lagerung bei 25°C/60%rF unter Stickstoffverschluss und in Aluminiumbeutel eingeschweißt. Die Nachtrocknung führt zu einer erhebliche Reduktion der Aggregatbildung für Pulver mit einem Mannitolanteil von 30% (w/w) und mehr.

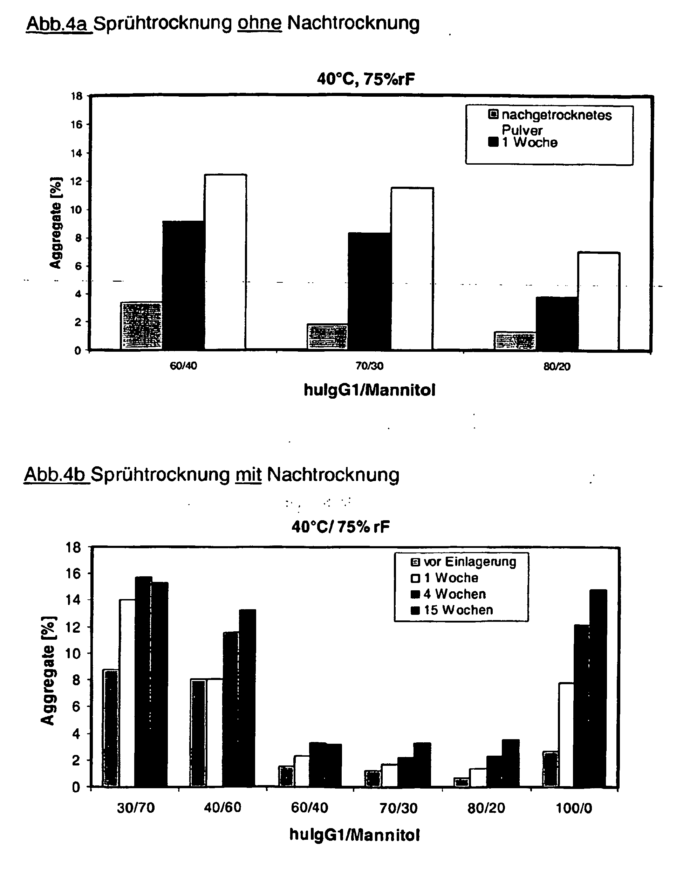

**Abbildung 4** zeigt die Aggregatbildung nach Sprühtrocknung mit und ohne anschließende Vakuumtrocknung jeweils vor der Einlagerung, nach 1, 4 und 15 Wochen Lagerung bei 40°C/75%rF unter Stickstoffverschluss und in Aluminiumbeutel eingeschweißt. Die Nachtrocknung führt zu einer erhebliche Reduktion der Aggregatbildung für Pulver mit einem Mannitolanteil von 30% (w/w) und mehr.

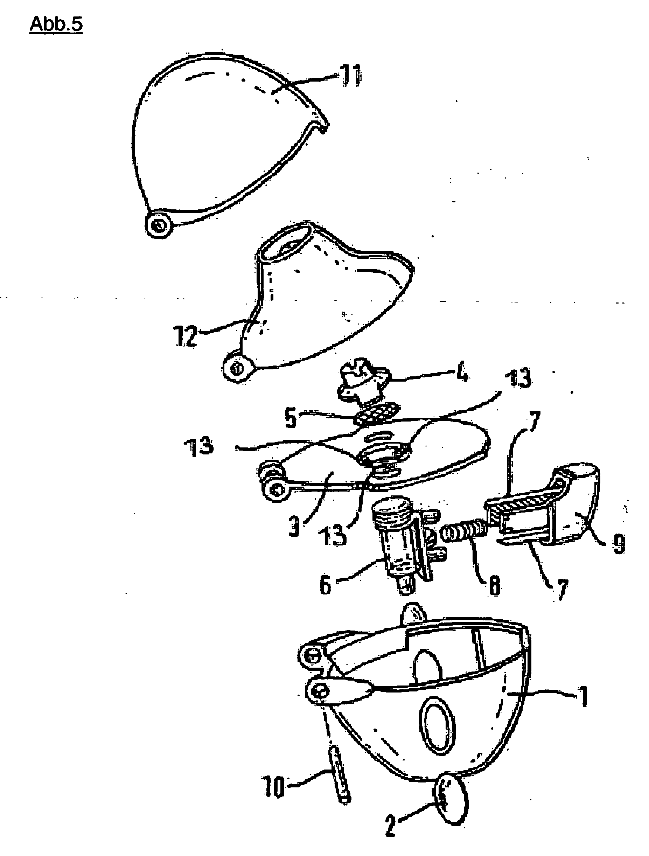

**Abbildung 5** zeigt einen Inhalator zur inhalativen Applikation von trockenen Pulverzubereitungen.

### Detaillierte Beschreibung der Erfindung

Die gegenwärtige Erfindung beruht auf der Tatsache, dass sich pharmazeutische Wirkstoffe, insbesondere biologische Makromoleküle, mit Hilfsstoffen sprühtrocknen und auf diese Weise lagerstabile inhalierbare Pulver herstellen lassen.

### Definitionen

Der Begriff "trockene Pulver" bezieht sich auf eine Zusammensetzung aus fein dispergierten festen Partikeln die frei fließend sind. Der Begriff "trocken" meint in diesem Zusammenhang, dass die Pulver über eine geringe Restfeuchte von unter 10%, gewöhnlich von unter 5%, und bevorzugt von unter 3% verfügen.

Der Begriff "amorph" meint, dass die pulverförmigen Formulierung weniger als 10% kristalline Anteile enthalten, vorzugsweise weniger als 7%, weiter bevorzugt weniger als 5%, insbesondere weniger als 4, 3, 2, oder 1 %.

Der Begriff "inhalierbar" meint, dass die Pulver zur pulmonalen Applikation geeignet sind. Inhalierbare Pulver lassen sich mit Hilfe eines Inhalationsgerätes dispergieren und inhalieren, so dass die Partikel die Lunge erreichen und gegebenenfalls über die Alveolen systemische Wirkung entfalten können. Inhalierbare Partikel weisen beispielsweise eine mittlere Teilchengröße zwischen 0,4-10µm (MMD = Mass median diameter), meistens zwischen 0,5-4 µm, bevorzugt zwischen 1-3 µm und/oder einen mittleren aerodynamischen Teilchendurchmesser (MMAD = Mass median aerodynamic diameter) zwischen 0,5-10 µm, vorzugsweise zwischen 0,5-7,5 µm, weiter bevorzugt zwischen 1-5 µm, noch weiter bevorzugt 1,5-4,5 µm und in besonders bevorzugter Weise zwischen 1,5-4 µm auf.

Der Begriff "dispergierbar" bezieht sich auf die Aerolisierung des Pulvers und meint, dass die ausgebrachte Menge größer als 30%, vorzugsweise größer als 40%, und in bevorzugter Weise größer als 50% ist. In einer weiter bevorzugten Ausführungsform beträgt der Anteil an ausgebrachter Menge mehr als 60%, vorzugsweise mehr als 70%, weiter bevorzugt mehr als 75%, nochweiter bevorzugt mehr als 80%.

Der Begriff "mittlere Teilchengröße" oder "mittlerer Teilchendurchmesser" bezieht sich auf den mittleren volumetrischen Durchmesser und gibt die volumetrische Teilchengröße an, bei der 50% der Teilchen des Pulvers einen kleineren volumetrischen Durchmesser aufweisen. Dieser entspricht somit dem mass median diameter (MMD) Zur Bestimmung der mittleren Teilchengröße dient im Zweifel die in dieser Patentschrift angegebene Methode (vgl. hierzu: Kapitel BEISPIELE, Methode).

Der Begriff "mittlerer aerodynamischer Teilchendurchmesser" (= mass median aerodynamic diameter (MMAD)) gibt die aerodynamische Teiclhengröße an, bei der 50% der Teilchen des Pulvers einen kleineren aerodynamischen Durchmesser aufweisen. Als Referenzmethode zur Bestimmung des MMAD dient im Zweifel die in dieser Patentschrift angegebene Methode (vgl. hierzu: Kapitel BEISPIELE, Methode).

Der Begriff "*Fine Particle Fraction*" (FPF) beschreibt den inhalierbaren Teil eines Pulvers bestehend aus Teilchen mit einer Teilchengröße von ≤ 7,5 µm MMAD. In gut dispergierbaren Pulver beträgt die FPF mehr als 20%, vorzugsweise mehr als 30%, besonders bevorzugt mehr als 40%, noch weiter bevorzugt mehr als 45%, noch weiter bevorzugt mehr als 50%.

Unter einem "pharmazeutischen Wirkstoff" ist eine Substanz, ein Arzneimittel, eine Zusammensetzung oder eine Kombination hieraus zu verstehen, die einen pharmakologischen, zumeist positiven, Effekt auf einen Organismus, ein Organ, und/oder eine Zelle ausübt, wenn der Wirkstoff mit dem Organismus, Organ oder der Zelle in Kontakt gebracht wird. Eingebracht in einen Patienten, kann der Effekt zu einem lokal oder systemisch sein.

Der Begriff "biologisches Makromolekül" meint Peptide, Proteine, Fette, Fettsäuren, oder auch Nukleinsäuren.

Mit dem Begriff "Peptid" oder "Polypeptid" sind Polymere von Aminosäuren bestehend aus zwei bis hundert Aminosäureresten gemeint. Der Begriff Peptid oder Polypeptid wird als pseudonym verwendet und umfasst sowohl Homo- als auch Heteropeptide, also Polymere von Aminosäuren bestehend aus einer oder aus verschiedenen Aminosäureresten.

Der Begriff "Protein" bezieht sich auf Polymere von Aminosäuren mit mehr als hundert Aminosäureresten. Der Begriff Protein umfasst hierbei sowohl einzelkettige Polymere als auch mehrkettige Polymere, wie z.B. Antikörper. Dieser Ausdruck schließt auch Proteine ein, die posttranslational durch Reaktionen wie beispielsweise Glykosylierung, Phosphorylierung, Acetylierung oder Proteinprozessierung modifiziert werden. Die Struktur des Polypeptids kann z.B. durch Substitutionen, Deletionen oder Insertionen von Aminosäuren, Fusion mit anderen Proteinen, unter Beibehaltung seiner biologischen Aktivität modifiziert werden. Der Ausdruck Protein schließt somit beispielsweise auch Fusionsproteine bestehend aus einem Immunglobulinanteil, z.B. dem Fc-Anteil, und einem Wachstumsfaktor, z.B. einem Interleukin, mit ein.

Der Begriff "native Konformation" meint, dass das Peptid/Protein nach Sprühtrocknung und/oder Lagerung seine ursprüngliche Sekundär- und Tertiärstruktur beibehält bzw. seine ursprüngliche biologische Aktivität nahezu unverändert bleibt.

Der Begriff "Hilfsstoff" meint einen Bestandteil, der die Stabilität des pharmazeutischen Wirkstoffs, insbesondere der biologischen Makromoleküle stabilisiert und/oder die Inhalierbarkeit der Pulver verbessert. Hilfsstoffe im Sinne der Erfindung können sein: Zucker, Polyole, Polymere oder eine Kombination aus den genannten Stoffen. Die Zucker sind Mono-, Di-, Oligo- oder Polysaccharide oder eine Kombination hiervon. Ferner fallen unter den Begriff Hilfsstoff: Aminosäuren, Di-, Tri-, Oligo- oder auch Polypeptide sowie Proteine, anorganische Salze, organische Salze oder Säuren und oberflächen aktive Substanzen.

Unter "Matrixbildnern" sind Hilfsstoffe zu verstehen, die im wesentlichen die Gestalt und Eigenschaft der Partikel mitbestimmen Matrixbildner können grundsätzlich Zucker, Polyole, Polymere, Aminosäuren, Di-, Tri-, Oligo-, Polypeptide, Proteine, oder auch Salze sein.

Der Begriff "Aggregate" bezieht sich auf nichtkovalent gebundene Di-, Tri-, Oligomere oder auf aus mehreren Molekülen bestehende Zusammenlagerungen. Der Anteil an Aggregate in den erfindungsgemäßen Pulvern sollte bei weniger als 5%, vorzugsweise bei weniger als 3,5%, besonders bevorzugt unterhalb von 2,5% liegen.

Der Ausdruck "enthaltend" meint auch die Ausführungsform "bestehend aus" ohne dies gesondert zu erwähnen. "Enthaltend einen Matrixbildner und einen pharmazeutischen Wirkstoff", mein somit auch die Ausführungsform "bestehend aus einem Matrixbildner und einem pharmazeutischen Wirkstoff".

### Pulverzusammensetzung

Die vorliegende Erfindung basiert auf der Beobachtung, dass sich vorteilhafte amorphe Pulver durch Sprühtrocknung mit anschließender Nachtrocknung herstellen lassen, wobei die sprühgetrockneten Pulver **(a)** zumindest einen pharmazeutischen Wirkstoff, vorzugsweise ein biologisches Makromolekül wie z.B. Peptide oder Proteine, und **(b)** zumindest einen Matrixbildner, vorzugsweise ein Polymer, Zucker, Zuckeralkohol (=Polyol) wie z.B. Mannitol, oder eine Kombination aus diesen, enthalten. Polymere, Zucker und deren Alkohole eignen sich besonders gut als Matrixbildner bei der Herstellung der erfindungsgemäßen sprühgetrockneten Pulvern, da sie zu gut dispergierbaren Pulvern führen und über ausgezeichnete stabilisierende Eigenschaften verfügen.

Geeignete Polymere umfassen beispielsweise Polyvinylpyrrolidone, derivatisierte Zellulosen, wie z.B. Hydroxymethyl-, Hydroxyethyl-, oder Hydroxypropyl-ethylzellulose, polymere Zucker wie z.B. Ficoll, Stärke wie z.B. Hydroxyethyl- oder Hydroxypropylstärke, Dextrine wie z.B. Cyclodextrine (2-Hydroxypropyl-βcyclodextrin, Sulfobutylether-β-cyclodextrin), Polyethylene, Glykole, Chitosan, Collagen, Hyaluronsäure, Polyacrylate, Polyvinylalkohole, Guar und/oder Pektine. Bei dem Zucker handelt es sich vorzugsweise um ein Mono-, Di-, Oligo- oder Polysaccharid oder um eine Kombination hieraus. Beispiele für Einfachzucker sind Fruktose, Maltose, Galaktose, Glucose, D-Mannose, Sorbose und der gleichen. Geeignete Zweifachzucker im Sinne der Erfindung sind beispielsweise Laktose, Sucrose, Trehalose, Cellobiose, und der gleichen. Als Mehrfachzucker oder Polysaccharide eignen sich insbesondere Raffinose, Melezitose, Dextrin, Stärke und der gleichen. Als Zuckeralkohole kommen neben Mannitol (bevorzugt), Xylitol, Maltitol, Galaktitol, Arabinitol, Adonitol, Laktitol, Sorbitol (Glucitol), Pyranosylsorbitol, Inositol, Myoinositol und der gleichen als Matrixbildner in Betracht.

Überraschenderweise wurde gefunden, dass entsprechende sprühgetrocknete Pulver besonders vorteilhafte Eigenschaften aufweisen, wenn ihr Restwassergehalt (= Restfeuchte) bei weniger als 1,2% (w/w), vorzugsweise bei weniger als 1,0% (w/w) liegt. Hohe Restfeuchten senken die Glasübergangstemperatur (Tg) einer amorphen Matrix und begünstigen so die Bildung von teilkristallinen oder vollständig kristallinen Strukturen. Überschreitet die Lagertemperatur den Glasübergang, so geht die amorphe Matrix in einen gummiartigen Zustand mit erhöhter Molekülmobilität und Reaktivität über.

Demnach betrifft die vorliegende Erfindung auch sprühgetrocknete amorphe Pulver enthaltend **(a)** zumindest einen pharmazeutischen Wirkstoff, vorzugsweise ein biologisches Makromolekül wie z.B. Peptide oder Proteine, u n d **(b)** zumindest einen Matrixbildner, vorzugsweise ein Polymer, ein Zucker, Zuckeralkohol (= Polyol) wie z.B. Mannitol, oder eine Kombination aus diesen, *dadurch gekennzeichnet,* dass der Restwassergehalt des Pulver bei ≤ 1,2% (w/w), vorzugsweise bei ≤ 1,0% (w/w) liegt, und wobei das Pulver als einen oder mehrere zusätzliche Hilfsstoffe Tripeptide der allgemeinen Formel Ile-X-X, X-Ile-X, X-X-Ile enthält, wobei X eine der folgenden Aminosäuren sein kann: Alanin, Glycin, Arginin, Histidin, Glutaminsäure, Glutamin, Asparagin, Asparaginsäure, Cystein, Leucin, Lysin, Isoleucin (Ile), Valin, Tryptophan, Methionin, Phenylalanin, Prolin, Serin, Threonin, Tyrosin, L-Aspartyl-L-phenylalanin-methylester (=Aspartam), Trimethylammonio-acetat und wobei Ile für Isoleucin steht.

Hohe Sprühtemperaturen stellen jedoch Probleme hinsichtlich der Denaturierung und Aggregation von pharmazeutischen Wirkstoffen, insbesondere von Peptiden/Proteinen dar. Es besteht somit eine gewisse Limitierung bei der Reduktion des Restwassergehaltes (= Restfeuchte) während des Sprühvorgangs. Je komplexer ein Peptid/Protein aufgebaut ist, desto labiler ist es gegenüber Wärme-/Hitzeeinwirkung. So sind kleine Peptide oder einzelkettige Proteine beispielsweise wesentlich hitzeresistenter als Antikörper. Es ist von daher schwierig eine richtige Balance zwischen der Menge an Hilfsstoff und der verwendeten Sprühtrocknungstemperatur zu finden. Hilfsstoffe üben auf der einen Seite einen stabilisierenden Effekt auf den Wirkstoff aus und lassen höhere Sprühtrocknungstemperaturen zu. Sie können aber andererseits aufgrund ihrer hygroskopen Eigenschaft die Aufnahme von Feuchtigkeit begünstigen, was wiederum die Kristallisation des Wirkstoffs zur Folge hat. Aus diesem Grund weisen die meisten sprühgetrockneten Pulver, insbesondere wenn sie komplexe Peptide/Protein als pharmazeutische Wirkstoffe enthalten, Restfeuchten von ungefähr 2% (w/w) oder sogar darüber auf (US 6,165,463; US 6,019,968; Maa et al., 1998, *supra*). Diese Restfeuchten sind hinsichtlich der Dispergierbarkeit und Langzeitstabilität von Nachteil.

Es war bekannt, dass sich insbesondere Zucker und deren Alkohole zur Stabilisierung von Proteinen eignen. Aufgrund ihr hygroskopischen Eigenschaften erweisen sich Zucker sowie deren Alkoholen bislang lediglich in Konzentrationen bis 20% (w/w) als wirklich vorteilhaft (vgl. Maa et al., 1998, *supra*). Es sind zwar Pulverzubereitung mit höherem Zucker-, Zuckeralkoholanteil beschrieben, diese zeigen jedoch aufgrund ihrer relativ hohen Restfeuchten nur eine eingeschränkte Dispergierbarkeit und Langzeitstabilität. Mit der vorliegenden Erfindung werden erstmals Pulver zur Verfügung gestellt, die die vorteilhaften Eigenschaften von Zucker und/oder Zuckalkoholen bei der Wirkstoffstabilisierung in Konzentrationen von größer 20% (w/w), vorzugsweise von 30-60% (w/w), besonders bevorzugt von 30-50% (w/w), insbesondere von 30-40% (w/w) ausnutzen. Demnach betrifft die vorliegende Erfindung sprühgetrocknete Pulver enthaltend **(a)** zumindest einen pharmazeutischen Wirkstoff und **(b)** zumindest eine Matrixbildner *dadurch gekennzeichnet,* dass es sich bei dem Matrixbildner um Zucker, Zuckeralkohole (= Polyole) und/oder eine Kombination aus diesen handelt wobei das Pulver eine Restfeuchte von weniger als 1.2%(w/w) aufweist und wobei das Pulver als einen oder mehrere zusätzliche Hilfsstoffe Tripeptide der allgemeinen Formel Ile-X-X, X-Ile-X, X-X-Ile enthält, wobei Ile für Isoleucin steht. Besonders bevorzugt sind solche Pulver, die Polyole, wie z.B. Mannitol als Matrixbildner enthalten.

Darüber hinaus sind solche Zucker und/oder Zuckeralkohole geeignet, die in der eingesetzten Konzentration einen Tg-Wert von größer 40°C, vorzugsweise von größer 45°C, noch mehr bevorzugt von größer 50°C, beispielsweise von größer 55, 60, 65, 70, 75, 80, 85, 90°C, etc. aufweisen. Folglich betrifft die vorliegende Erfindung in einer weiteren Ausführungsform auch amorphe sprühgetrocknete Pulver enthaltend, **(a)** zumindest einen pharmazeutischen Wirkstoff, vorzugsweise ein biologisches Makromolekül wie z.B. Peptide oder Proteine, u n d **(b)** zumindest einen Matrixbildner, vorzugsweise ein Zucker oder Zuckeralkohol (= Polyol) wie z.B. Mannitol, *dadurch gekennzeichnet,* dass der Tg des Pulvers oberhalb von 40°C, vorzugsweise oberhalb von 45°C, weiter bevorzugt oberhalb von 50°C, noch weiter bevorzugt oberhalb von 55°C, beispielsweise oberhalb von 60, 65, 70, 75, 80, 85, 90°C, liegt. Besonders bevorzugt ist ein entsprechendes Pulver, das Mannitol als Matrixbildner enthält. Der Tg eines Pulvers lässt sich experimentell mittels DSC bestimmen (DSC = *Differential Scanning Calorimetry*) (Breen et al., 2001, Pharm. Res., 18(9), 1345-1353). Hierbei wird die Zunahme der Wärmekapazität in Abhängigkeit von der Temperatur aufgezeichnet. Da globuläre Proteine nur geringe Änderungen in der Wärmekapazität am Tg zeigen, werden die Tg-Werte mittels modulierender DSC gemessen.

Im Allgemeinen beträgt der Anteil des entsprechenden Matrixbildners in den erfindungsgemäßen Pulvern zwischen 20-60% (w/w), besonders bevorzugt zwischen 20-40% (w/w) der Trockenmasse des Pulvers. Nach einer weiter bevorzugten Ausführungsform der Erfindung beträgt der Anteil des entsprechenden Matrixbildners, z.B. der Polyol- oder Mannitolanteil mehr als 20% (w/w) der Trockenmasse des Pulvers, vorzugsweise zwischen 30-60% (w/w), besonders bevorzugt zwischen 30-50% (w/w), noch weiter bevorzugt zwischen 30-40% (w/w) und noch weiter bevorzugt zwischen 35-40% (w/w). Wie aus den beigefügten Beispielen ersichtlich wird, führt die Erhöhung des Matrixanteils auf über 20% (w/w), vorzugsweise auf 30-60% (w/w) bei hoher Dispergierbarkeit zu keiner wesentlichen Zunahme an Aggregaten. Entsprechend dieser Ausführungsformen, kann der Anteil an dem entsprechenden Matrixbildner von daher ungefähr 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 oder 60% (w/w) der Trockenmasse des Pulvers betragen. Die entsprechenden Ausführungsformen gelten insbesondere für Pulver, bei denen Polyole, insbesondere bei denen Mannitol als Matrixbildner verwendet wird.

Nach einer weiter bevorzugten Ausführungsform enthält das erfindungsgemäße Pulver lediglich einen der oben aufgeführten Zucker, Polyole oder Polymere als Matrixbildner in einer der angegebenen Konzentrationen, wobei den erfindungsgemäßen Pulvern weitere Zucker, Polyole oder Polymere in geringeren Mengen bis 20% (w/w), vorzugsweise bis 10% (w/w), besonders bevorzugt bis 5% (w/w) zugemischt werden können. Vorzugsweise handelt es sich bei dem Matrixbildner um Mannitol in einer der oben angegebenen Konzentration.

Nach einer weiteren erfindungsgemäßen Ausführungsform beträgt der Gesamtanteil an Zucker, Polyol und/oder Polymer im Pulver maximal 99% (w/w), vorzugsweise maximal 95% (w/w), weiter bevorzugt maximal 90% (w/w), noch weiter bevorzugt maximal 80% (w/w). In einer weiter bevorzugten Ausführungsform beträgt der Zucker-, Polyol- und/oder Polymeranteil im Pulver maximal 70% (w/w), vorzugsweise maximal 60% (w/w), besonders bevorzugt maximal 50% (w/w) noch weiter bevorzugt zwischen 40 und 30% (w/w). Die Kombination aus geringer Restfeuchte - wie oben beschrieben - und der Verwendung von entsprechenden Matrixbildnern - wie oben angegeben - führte überraschenderweise zu lagerstabilen Pulvern mit hoher Dispergierbarkeit. Der Anteil an Aggregaten lag nach Sprühtrocknung und Nachtrocknung in den erfindungsgemäßen Pulvern mit einem Matrixanteil zwischen 20-60% bei weniger als 2%. Selbst nach einer Lagerung von 15 Wochen bei 30 bzw. 40°C und einer relativen Feuchte von 60% bzw. 75% stieg der Anteil an Aggregaten nicht über 3,5%. Pulver mit höheren Restfeuchten wiesen vergleichsweise einen hohen Anteil an Aggregaten auf (nach Lagerung von nur 4 Wochen bei 40°C und 75% rF zwischen 7 und 13%). Diese Werte decken sich auch mit den Beobachtungen von Maa et al., 1998, *supra*.

Die vorliegenden erfindungsgemäßen Pulver können zusätzlich Salze, vorwiegend pharmazeutisch akzeptable Salze beinhalten. Hierbei handelt es sich beispielsweise um anorganische Salze wie Chloride, Sulfate, Phosphate, di-Phosphate, Hydrobromide und/oder Nitrat-Salze. Ferner können die erfindungsgemäßen Pulver auch organische Salze beinhalten, wie z.B. Malate, Maleate, Fumarate, Tartrate, Succinate, Ethylsuccinate, Citrate, Acetate, Lactate, Methansulfonate, Benzoate, Ascorbate, Paratoluensulfonate, Palmoate, Salicylate, Stearate, Estolate, Gluceptate oder Labionat-Salze. Gleichzeitig können entsprechende Salze pharmazeutisch akzeptable Kationen, wie beispielsweise Natrium, Kalium, Calzium, Aluminium, Lithium oder Ammonium enthalten. Besonders bevorzugt ist die Verwendung von entsprechenden Kationen in Verbindung mit der Stabilisierung von Proteinen sowie ein Pulver enthaltend Mannitol in Kombination mit Citrat. Geeignet sind insbesondere Citratkonzentrationen zwischen 0,1 und 150 mM (bezieht sich auf die zu versprühende Lösung).

Nach einer weiteren erfindungsgemäßen Ausführungsform können die Pulver zusätzlich oberflächenaktiven Substanzen wie Tween 20, 40, 60, 80, Brij 35, Pluronic F 88 und Pluronic F 127 enthalten. Diese werden vorzugsweise in einer Konzentration von 0,01-0,1% (w/w) eingesetzt. Besonders bevorzugt ist ein sprühgetrocknetes Pulver, dass als Matrixbildner einem Zucker oder ein Polyol, insbesondere Mannitol und zusätzlich Tween 20, vorzugsweise in einer Konzentration von 0,01-0,1% (w/w), als oberflächenaktive Substanz enthält. Pulver mit einem Anteil von ungefähr 29.95% (w/w) Mannitol, 69,95% (w/w)eines Antikörpers und 0,1% (w/w) Tween 20 wiesen nach Langzeitlagerung einen Aggregatanteil von weniger als 1 % auf und erwiesen sich als besonders vorteilhaft im Sinne der Erfindung.

Die erfindungsgemäßen Pulver können darüber hinaus weitere Hilfsstoffe, wie beispielsweise Aminosäure, Peptide, nicht-biologische oder biologische Polymere, und/oder einen oder mehrere der oben aufgeführten Zucker enthalten. Weitere, im Stand der Technik bekannte Hilfsstoffe sind beispielsweise Lipide, Fettsäuren Fettsäureester, Steroide (z.B. Cholesterol) oder Chelatbildner (z.B. EDTA) sowie diverse Kationen (siehe oben). Besonders bevorzugt sind Hilfsstoffe mit einer hohen Glasübergangstemperatur, beispielsweise von größer 40°C, vorzugsweise von größer 45°C, oder von größer 55°C. Eine Auflistung geeigneter Hilfsstoffe findet sich beispielsweise in Kippe (Eds.), "Handbook of Pharmaceutical Excipients" 3rd Ed., 2000.

Geeignete proteinhaltige Hilfsstoffe sind beispielsweise Albumin (humanen order rekombinanten Ursprungs), Gelatine, Kasein, Hämoglobin und der gleichen. Geeignete Aminosäuren umfassen beispielsweise Alanin, Glycin, Arginin, Histidin, Glutamat, Asparagin, Cystein, Leucin, Lysin, Iso-Leucin, Valin, Tryptophan, Methionin, Phenylalanin, Tyrosin, L-Aspartyl-L-phenylalanin-methylester (= Aspartam), Trimethylammonioacetat (= Betain) und der gleichen. Vorzugsweise werden solche Aminosäuren verwendet, die als Puffer (z.B. Glycin oder Histidin) und/oder als dispergierendes Agens wirken. Unter die letzten Gruppen fallen insbesondere vorwiegend hydrophobe Aminosäuren, wie z.B. Leucin, Valin, Iso-Leucin, Tryptophan, Alanin, Methionin, Phenylalanin, Tyrosin, Histidin, oder Prolin.

Im Rahmen der vorliegenden Erfindung erweist sich insbesondere die Verwendung von Isoleucin als zusätzlichen Hilfsstoff als vorteilhaft. Besonders vorteilhaft ist die Verwendung von Isoleucin in einer Konzentration von 1 bis 19,99% (w/w), vorzugsweise von 5 bis 19,99% (w/w), noch weiter bevorzugt von 10 bis 19,99% (w/w). Der Anteil an kann allerdings auch auf Werte bis 30% (w/w) erhöht werden, sofern der Anteil an dem Matrixbildner oder der Anteil an dem pharmazeutischen Wirkstoff entsprechend reduziert wird, so dass der Feststoffgehalt des Pulvers maximal 100% (w/w) ergibt.

Vorteilhaft ist auch die Verwendung von Di-, Tri-, Oligo-, oder Polypeptiden als Hilfsstoff, die eine oder mehrere dieser vornehmlich hydrophoben Aminosäurereste beinhalten. Geeignete Beispiele für Di- oder Tri-Peptide finden sich u.a. in WO 01/32144, auf die an dieser Stelle inhaltlich Bezug genommen wird. Hierbei handelt es sich beispielsweise um ein oder mehrere der nachfolgenden Tri-Peptide: Leu-Leu-Gly, Leu-Leu-Ala, Leu-Leu-Val, Leu-Leu-Leu, Leu-Leu-Met, Leu-Leu-Pro, Leu-Leu-Phe, Leu-Leu-Trp, Leu-Leu-Ser, Leu-Leu-Thr, Leu-Leu-Cys, Leu-Leu-Tyr, Leu-Leu-Asp, Leu-Leu-Glu, Leu-Leu-Lys, Leu-Leu-Arg, Leu-Leu-His, Leu-Gly-Leu, Leu-Ala-Leu, Leu-Val-Leu, Leu-Met-Leu, Leu-Pro-Leu, Leu-Phe-Leu, Leu-Trp-Leu, Leu-Ser-Leu, Leu-Thr-Leu, Leu-Cys-Leu, Leu-Tyr-Leu, Leu-Asp-Leu, Leu-Glu-Leu, Leu-Lys-Leu, Leu-Arg-Leu und Leu-His-Leu. Als besonders vorteilhaft hat sich die Verwendung von Tri-Peptiden der allgemeinen Formeln: Ile-X-X; X-Ile-X; X-X-Ile erwiesen, wobei X eine der folgenden Aminosäuren sein kann: Alanin, Glycin, Arginin, Histidin, Glutaminsäure, Glutamin, Asparagin, Asparaginsäure, Cystein, Leucin, Lysin, Isoleucin (Ile), Valin, Tryptophan, Methionin, Phenylalanin, Prolin, Serin, Threonin, Tyrosin, L-Aspartyl-L-phenylalanin-methylester (= Aspartam), Trimethylammonio-acetat und Ile für Isoleucin steht. Besonders bevorzugt sind entsprechende Tri-Peptide der Formel (Ile)₂-X, beispielsweise Ile-Ile-X, Ile-X-Ile, oder X-Ile-Ile, wobei X wiederum eine der oben aufgeführten Aminosäuren sein kann. Hierunter fallen beispielsweise die Tri-Peptide: Ile-Ile-Gly, Ile-Ile-Ala, Ile-Ile-Val, Ile-Ile-Ile, Ile-Ile-Met, Ile-Ile-Pro, Ile-Ile-Phe, Ile-Ile-Trp, Ile-Ile-Ser, Ile-Ile-Thr, Ile-Ile-Cys, Ile-Ile-Tyr, Ile-Ile-Asp, Ile-Ile-Glu, Ile-Ile-Lys, Ile-Ile-Arg, Ile-Ile-His, Ile-Gly-Ile, Ile-Ala-Ile, Ile-Val-Ile, Ile-Met-Ile, Ile-Pro-Ile, Ile-Phe-Ile, Ile-Trp-Ile, Ile-Ser-Ile, Ile-Thr-Ile, Ile-Cys-Ile, Ile-Tyr-Ile, Ile-Asp-Ile, Ile-Glu-Ile, Ile-Lys-Ile, Ile-Arg-Ile, Ile-His-Ile. Besonders vorteilhaft ist die Verwendung von Ile-Ile-Ile.

Nach einer weiteren Ausführungsform betrifft die vorliegenden Erfindung folglich sprühgetrocknete Pulver, die in Bezug auf ihre Trockenmasse a) einen Matrixbildner, vorzugsweise ein Polyol wie zum Beispiel Mannitol in einer Konzentration von 20-60% (w/w), besonders bevorzugt in einer Konzentration von 25-50% (w/w), noch weiter bevorzugt in einer Konzentration von 30-40% (w/w) b) zwischen 1 und 19,99% (w/w) Aminosäuren und c) zwischen 0,01 und 79% (w/w), vorzugsweise zwischen 0,01 und 69% (w/w) eines pharmazeutischen Wirkstoffs, vorzugsweise eines Peptids/Proteins, beispielsweise eines Antikörpers, enthalten, wobei die Summe der Gewichtsanteile maximal 100% (w/w) beträgt. Folglich betrifft die vorliegende Erfindung nach einer weiteren Ausführungsform auch Pulver, die beispielsweise 20% (w/w) Matrixbildner, vorzugsweise Polyol wie zum Beispiel Mannitol / 10% (w/w) Aminosäure / 70% (w/w) pharmazeutischen Wirkstoff, vorzugsweise ein Peptid/Protein, beispielsweise einen Antikörper, (20/10/70); oder (21/10/69) beispielsweise (22/10/68); (23/10/67); (24/10/66); (25/10/65); (26/10/64); (27/10/63); (28/10/62); (29/10/61); (30/10/60); (31/10/59); (32/10/58); (33/10/57); (34/10/56); (35/10/55); (36/10/54); (37/10/53); (38/10/52); (39/10/51) oder (40/10/50) oder enthalten oder aus diesen bestehen. Sofern der Aminosäureanteil bei einem konstanten Anteil von beispielsweise 20% (w/w) Mannitol zwischen 1 und 20% (w/w) variiert, beispielsweise 1, 2, 3 ... 8, 9, 10, 11, 12, 13 ... 18, 19, 19,1, 19,2, 19,3 ... 19,8, 19,9, 19,91, 19,92, 19,93, ... 19,97, 19,98, 19,99% (w/w) beträgt, so verringert sich der Anteil an pharmazeutischen Wirkstoff, vorzugsweise eines Peptids/Proteins, beispielsweise eines Antikörpers ausgehend von 79% (w/w) auf 78, 77, ... 72, 71, 70, 69, 68, 67, ... 63, 62, 61, 60,9 , 60,8, 60,7 ... 60,3, 60,2, 60,1, 60,09, 60,08, ... 60,03, 60,02, 60,01% (w/w). Sofern sich der Anteil an Matrixbildner bei konstanten Anteil an pharmazeutischen Wirkstoff von beispielsweise 60% (w/w) ausgehend von 20 auf 39% (w/w) erhöht, beispielsweise auf 21, 22, 23, 24, 25, 26, 27, 28, 29, 30, 31, 32, 33, 34, 35, 36, 37, 38, 38,1, 38,2, 38,3 ... 38,8, 38,9, 38,91, 38,92 ... 38,97, 38,98, 38,99, 39% (w/w), reduziert sich der Aminosäureanteil ausgehend von 20 auf 1% (w/w), beispielsweise auf 19, 18, 17, ... 13, 12,11, 10, 9, 8, 7, ... 3, 2, 1,9, 1,8, 1,7 ... 1,3, 1,2, 1,1 1,09, 1,08, ... 1,03, 1,02, 1,01, 1% (w/w), so dass die Summe der Gewichtsanteile der einzelnen Pulverbestandteile in Bezug auf die Trockenmasse des Pulvers maximal 100% (w/w) ergibt. Als besonders vorteilhaft erweist sich hierbei die Verwendung von Mannitol als Matrixbildner im Bereich von 21 bis 50% (w/w), vorzugsweise von 25 bis 50% (w/w), weiter bevorzugt von 30 bis 40% (w/w). Durch Zusatz von weiteren Hilfsstoffen oder Salzen kann der Anteil an Mannitol, Aminosäuren und/oder pharmazeutischem Wirkstoff entsprechend angepasst/reduziert werden, so dass die Gewichtsanteile der einzelnen Bestandteile in der Summe maximal 100% (w/w) ergibt.

Handelt es bei der zugesetzten Aminosäure um Isoleucin, so sind nach einer weiteren Ausführungsform Pulver erfindungsgemäß die **a)** einen Matrixbildner, vorzugsweise einem Polyol, beispielsweise Mannitol in einer Konzentration 20-60% (w/w) von besonders bevorzugt in einer Konzentration von 25-50% (w/w), noch weiter bevorzugt in einer Konzentration von 30-40% (w/w) **b)** zwischen 1 und 19,99% (w/w) Isoleucin und **c)** zwischen 0,01 und 79% (w/w), vorzugsweise zwischen 0,01 und 69% (w/w) eines pharmazeutischen Wirkstoffs, vorzugsweise eines Peptids/Proteins, beispielsweise eines Antikörpers, enthalten, wobei die Summe der Gewichtsanteile maximal 100% (w/w) beträgt. Folglich betrifft die vorliegende Erfindung nach einer weiteren Ausführungsform auch Pulver, die beispielsweise 20% (w/w) Matrixbildner, vorzugsweise ein Polyol wie zum Beispiel Mannitol / 10% (w/w) Isoleucin / 70% (w/w) pharmazeutischen Wirkstoff (20/10/70); oder beispielsweise (21/10/69); (22/10/68); (23/10/67); (24/10/66); (25/10/65); (26/10/64); (27/10/63); (28/10/62); (29/10/61); (30/10/60); (31/10/59); (32/10/58); (33/10/57); (34/10/56); (35/10/55); (36/10/54); (37/10/53); (38/10/52); (39/10/51) oder (40/10/50) enthalten oder aus diesen bestehen. Sofern der Isoleucinanteil bei einem konstanten Anteil an Matrixbildner, wie zum Beispiel Mannitol, von zumindest 20% (w/w), zwischen 1 und 20% (w/w) variiert, beispielsweise 1, 2, 3 ... 8, 9, 10, 11, 12, 13 ... 18, 19, 19,1, 19,2, 19,3 ... 19,8, 19,9, 19,91, 19,92, 19,93, ... 19,97, 19,98, 19,99% (w/w) beträgt, so verringert sich der Anteil an pharmazeutischen Wirkstoff, vorzugsweise einem Peptid/Protein ausgehend von 79% (w/w) auf 78, 77, ... 72, 71, 70, 69, 68, 67, ... 63, 62, 61, 60,9 , 60,8, 60,7 ... 60,3, 60,2, 60,1, 60,09, 60,08, ... 60,03, 60,02, 60,01 % (w/w). Sofern der Anteil an Matrixbildner wie zum Beispiel Mannitol, bei einem konstanten Anteil an pharmazeutischen Wirkstoff, vorzugsweise einen Protein/Peptid von 60% (w/w) ausgehend von 20 auf 39% (w/w), erhöht wird, beispielsweise auf 21, 22, 23, 24, 25, 26, 27, 28, 29, 30, 31, 32, 33, 34, 35, 36, 37, 38, 38,1, 38,2, 38,3 ... 38,8, 38,9, 38,91, 38,92 ... 38,97, 38,98, 38,99, 39 %(w/w), reduziert sich der Isoleucinanteil ausgehend von 20 auf 1% (w/w), beispielsweise auf 19, 18, 17, ... 13, 12,11, 10, 9, 8, 7, ... 3, 2, 1,9, 1,8, 1,7 ... 1,3, 1,2, 1,1 1,09, 1,08, ... 1,03, 1,02, 1,01, 1% (w/w), so dass die Summe der Gewichtsanteile der einzelnen Pulverbestandteile in Bezug auf die Trockenmasse des Pulvers maximal 100% (w/w) ergibt. Besonders vorteilhaft hat sich hierbei die Verwendung von Mannitol als Matrixbildner im Bereich von 21 bis 50% (w/w), vorzugsweise von 25 bis 50% (w/w), weiter bevorzugt von 30 bis 40% (w/w) erwiesen. Durch Zusatz von weiteren Hilfsstoffen oder Salzen kann der Anteil an Mannitol, Isoleucin und/oder pharmazeutischem Wirkstoff entsprechend angepasst/reduziert werden, so dass die Gewichtsanteile der einzelnen Bestandteile in der Summe maximal 100% (w/w) ergibt.

Darüber hinaus betrifft die vorliegende Erfindung nach einer weiteren Ausführungsform auch Pulver **a)** mit einem Matrixbildner, vorzugsweise einem Polyol, beispielsweise Mannitol in einer Konzentration von 20-60% (w/w), besonders bevorzugt in einer Konzentration von 25-50% (w/w), noch weiter bevorzugt in einer Konzentration von 30-40% (w/w) **b)** zwischen 1 und 19,99% (w/w) Di- oder Tri-Peptid, vorzugsweise mit einem oder mehreren Isoleucinresten und **c)** zwischen 0,01 und 79% (w/w), vorzugsweise zwischen 0,01 und 69% (w/w) eines pharmazeutischen Wirkstoffs, vorzugsweise eines Peptids/Proteins, beispielsweise eines Antikörpers, wobei die Summe der Gewichtsanteile maximal 100% (w/w) beträgt. Folglich betrifft die vorliegende Erfindung nach einer weiteren Ausführungsform auch Pulver, die beispielsweise 20% (w/w) Matrixbildner, vorzugsweise Polyol wie zum Beispiel Mannitol / 10% (w/w) Di- oder Tri-Peptid, vorzugsweise mit einem oder mehreren Isoleucinresten / 70% (w/w) pharmazeutischen Wirkstoff = (20/10/70); oder beispielsweise (21/1069); (22/10/68); (23/10/67); (24/10/66); (25/10/65); (26/10/64); (27/10/63); (28/10/62); (29/10/61); (30/10/60); (31/10/59); (32/10/58); (33/10/57); (34/10/56); (35/10/55); (36/10/54); (37/10/53); (38/10/52); (39/10/51) oder (40/10/50) enthalten oder aus diesen bestehen. Sofern der Anteil an besagten Di- oder Tri-Peptiden, bei einem konstanten Anteil an Matrixbildner, vorzugsweise Mannitol, von zumindest 20% (w/w), zwischen 1 und 20% (w/w) variiert, und beispielsweise 1, 2, 3 ... 8, 9, 10, 11, 12, 13 ... 18, 19, 19,1, 19,2, 19,3 ... 19,8, 19,9, 19,91, 19,92, 19,93, ... 19,97, 19,98, 19,99% (w/w) beträgt, so verringert sich der Anteil an pharmazeutischen Wirkstoff, vorzugsweise an Peptid/Protein, beispielsweise an einem Antikörper ausgehend von 79% (w/w) auf 78, 77, ... 72, 71, 70, 69, 68, 67, ... 63, 62, 61, 60,9 , 60,8, 60,7 ... 60,3, 60,2, 60,1, 60,09, 60,08, ... 60,03, 60,02, 60,01% (w/w). Sofern der Anteil an Matrixbildner, vorzugsweise einem Polyol wie zum Beispiel Mannitol, bei einem konstanten Anteil an pharmazeutischen Wirkstoff von 60% (w/w) sich ausgehend von 20 auf 39% (w/w) erhöht, beispielsweise auf 21, 22, 23, 24, 25, 26, 27, 28, 29, 30, 31, 32, 33, 34, 35, 36, 37, 38, 38,1, 38,2, 38,3 ... 38,8, 38,9, 38,91, 38,92 ... 38,97, 38,98, 38,99, 39 %(w/w), reduziert sich der Anteil an Di- oder Tri-Peptid, vorzugsweise mit einem oder mehreren Isoleucinresten, ausgehend von 20 auf 1% (w/w), beispielsweise auf 19, 18, 17, ... 13, 12,11, 10, 9, 8, 7, ... 3, 2, 1,9, 1,8, 1,7 ... 1,3, 1,2, 1,1 1,09, 1,08, .... 1,03, 1,02, 1,01, 1% (w/w), so dass die Summe der Gewichtsanteile der einzelnen Pulverbestandteile in Bezug auf die Trockenmasse des Pulvers maximal 100% (w/w) ergibt. Besonders vorteilhaft erweist sich hierbei die Verwendung von Mannitol als Matrixbildner im Bereich von 21 bis 50% (w/w), vorzugsweise von 25 bis 50% (w/w), weiter bevorzugt von 30 bis 40% (w/w). Durch Zusatz von weiteren Hilfsstoffen oder Salzen kann der Anteil an Mannitol, Di- oder Tri-Peptid, vorzugsweise mit einem oder mehreren Isoleucinresten und/oder pharmazeutischem Wirkstoff entsprechend angepasst/reduziert werde, so dass die Gewichtsanteile der einzelnen Bestandteile in der Summe maximal 100% (w/w) ergibt.

Handelt es bei dem zugesetzten Tri-Peptid um Tri-Isoleucin, so sind nach einer weiteren Ausführungsform Pulver, erfindungsgemäß mit **a)** einem Matrixbildner, vorzugsweise einem Polyol, beispielsweise Mannitol in einer Konzentration von 20-60% (w/w), besonders bevorzugt in einer Konzentration von 25-50% (w/w), noch weiter bevorzugt in einer Konzentration von 30-40% (w/w) **b)** zwischen 1 und 19,99% (w/w) Tri-Isoleucin und **c)** zwischen 0,01 und 79% (w/w), vorzugsweise zwischen 0,01 und 69% (w/w) eines pharmazeutischen Wirkstoffs, vorzugsweise eines Peptids/Proteins wie zum Beispiel einen Antikörper, wobei die Summe der Gewichtsanteile maximal 100% (w/w) beträgt. Folglich betrifft die vorliegende Erfindung nach einer weiteren Ausführungsform auch Pulver, die beispielsweise 20% (w/w) Matrixbildner, vorzugsweise Polyol wie zum Beispiel Mannitol / 10% (w/w) Tri-Isoleucin / 70% (w/w) pharmazeutischen Wirkstoff (21/10/69); oder beispielsweise (22/10/68); (23/10/67); (24_{/}10/66); (25/10/65); (26/10/64); (27/10/63); (28/10/62); (29/10/61); (30/10/60); (31/10/59); (32/10/58); (33/10/57); (34/10/56); (35/10/55); (36/10/54); (37/10/53); (38/10/52); (39/10/51) oder (40/10/50) oder enthalten oder aus diesen bestehen. Sofern der Tri-Isoleucinanteil bei einem konstanten Anteil an Matrixbildner, wie zum Beispiel Mannitol, von zumindest 20% (w/w), zwischen 1 und 20% (w/w) variiert, beispielsweise 1, 2, 3 ... 8, 9, 10, 11, 12, 13 ... 18, 19, 19,1, 19,2, 19,3 ... 19,8, 19,9, 19,91, 19,92, 19,93, ... 19,97, 19,98, 19,99% (w/w) beträgt, so verringert sich der Anteil an pharmazeutischen Wirkstoff, vorzugsweise einem Peptid/Protein beispielsweise einem Antikörper ausgehend von 79% (w/w) auf 78, 77, ... 72, 71, 70, 69, 68, 67, ... 63, 62, 61, 60,9 , 60,8, 60,7 ... 60,3, 60,2, 60,1, 60,09, 60,08, ... 60,03, 60,02, 60,01% (w/w). Sofern sich der Anteil an Matrixbildner, vorzugsweise einem Polyol wie zum Beispiel Mannitol, bei einem konstanten Anteil an pharmazeutischen Wirkstoff, vorzugsweise einen Protein/Peptid von 60% (w/w), ausgehend von 20 auf 39% (w/w) erhöht, beispielsweise auf 21, 22, 23, 24, 25, 26, 27, 28, 29, 30, 31, 32, 33, 34, 35, 36, 37, 38, 38,1, 38,2, 38,3 ... 38,8, 38,9, 38;91, 38,92 ... 38,97, 38,98, 38,99, 39 %(w/w), reduziert sich der Tri-Isoleucinanteil ausgehend von 20 auf 1 % (w/w), beispielsweise auf 19, 18, 17, ... 13, 12,11, 10, 9, 8, 7, ... 3, 2, 1,9, 1,8, 1,7 ... 1,3, 1,2, 1,1 1,09, 1,08, ... 1,03, 1,02, 1,01, 1% (w/w), so dass die Summe der Gewichtsanteile der einzelnen Pulverbestandteile in Bezug auf die Trockenmasse des Pulvers maximal 100% (w/w) ergibt. Besonders vorteilhaft ist die Verwendung von Mannitol als Matrixbildner im Bereich von 21 bis 50% (w/w), vorzugsweise von 25 bis 50% (w/w), weiter bevorzugt von 30 bis 40% (w/w). Durch Zusatz von weiteren Hilfsstoffen oder Salzen kann der Anteil an Mannitol, Tri-Isoleucin und/oder pharmazeutischem Wirkstoff entsprechend angepasst/reduziert werden, so dass die Gewichtsanteile der einzelnen Bestandteile in der Summe maximal 100% (w/w) ergibt.

Nach einer weiteren Ausführungsform hat sich eine Kombination aus (a) Mannitol, vorzugsweise in einer Konzentration von 20-60% (w/w), besonders bevorzugt in einer Konzentration von 30-50% (w/w), noch weiter bevorzugt in einer Konzentration von 30-40% (w/w) und (b) Histidin oder Glycin u n d (c) einem pharmazeutischen Wirkstoff, vorzugsweise ein Protein oder Peptid, besonders bevorzugt ein Antikörper, als geeignet erwiesen. Als besonders vorteilhaft hat sich hierbei die Sprühtrocknung von Lösungen gezeigt die neben dem pharmazeutischen Wirkstoff und Mannitol 1,6 mM Glycin und 25 mM Histidin enthielten.

Geeignete Polymere umfassen beispielsweise die oben bereits als Matrixbildner erwähnte Polyvinylpyrrolidone, derivatisierte Zellulosen, wie z.B. Hydroxymethyl-, Hydroxyethyl-, oder Hydroxypropyl-ethylzellulose, polymere Zucker wie z.B. Fiscoll, Särke wie z.B. Hydroxyethyl- oder Hydroxypropylstärke, Dextrine wie z.B. Cyclodextrine (2-Hydroxypropyl-β-cyclodextrin, Sulfobutylether-β-cyclodextrin), Polyethylene, Glykole und/oder Pektine.

Bei dem pharmazeutischen Wirkstoff handelt es sich vorzugsweise um ein biologisches Makromolekül. Entsprechend der oben angegebenen Definition sind hierunter beispielsweise Peptide, Proteine, Fette, Fettsäuren, oder auch Nukleinsäuren zu verstehen.

Biopharmazeutisch bedeutsame Proteine/Polypeptide umfassen z.B. Antikörper, Enzyme, Wachstumsfaktoren z.B. Cytokine, Lymphokine, Adhäsionsmoleküle, Rezeptoren sowie deren Derivate bzw. Fragmente, sind aber nicht auf diese beschränkt. Im Allgemeinen sind alle Polypeptide bedeutsam, die als Agonisten oder Antagonisten wirken und/oder therapeutische oder diagnostische Anwendung finden können.

Geeignete Peptide oder Proteine im Sinne der Erfindung sind beispielsweise Insulin, Insulin-ähnlicher Wachstumsfaktor, humanes Wachstumshormon (hGH) und andere Wachstumsfaktoren, Gewebeplasminogenaktivator (tPA), Erythropoetin (EPO), Cytokine, beispielsweise Interleukine (IL) wie IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, Interferon (IFN)-alpha, beta, gamma, omega oder tau, Tumornekrosefaktor (TNF) wie z.B. TNFalpha, beta oder gamma, TRAIL, G-CSF, GM-CSF, M-CSF, MCP-1 und VEGF. Weitere Beispiele sind monoklonale, polyklonale, multispezifische und einzelkettige *(single chain*) Antikörper und Fragmente davon, wie z.B. Fab, Fab', F(ab')₂, Fc und Fc'-Fragmente, leichte (L) und schwere (H) Immunglobulinketten und deren konstante, variable oder hypervariable Regionen sowie Fv- und Fd-Fragmente (Chamov et al., 1999, Antibody Fusion Proteins, Wiley-Liss Inc.). Die Antikörper können humanen oder nicht-humanen Ursprungs sein. Hierunter fallen beispielsweise die im Menschen bekannten Klassen: IgA, IgD, IgE, IgG and IgM, mit ihren verschiedenen Subklassen, beispielsweise IgA1, IgA2 and IgG1, IgG2, IgG3 and IgG4. Auch humanisierte und chimäre Antikörper kommen in Frage. Von besonderer therapeutischer Bedeutung und somit Gegenstand der vorliegenden Erfindung sind Pulverformulierungen die Antikörper gegen beispielsweise verschiedene Oberflächenantigene wie CD4, CD20 oder CD44, verschiedene Cytokine, beispielsweise IL2, IL4 oder IL5. Weitere Beispiele sind Antikörper gegen bestimmte Immunglobulin-Klassen (z.B. anti-IgE-Antikörper) oder gegen virale Proteine (zb. anti-RSV-, anti-CMV Antikörper, etc.).

Fab-Fragmente (Fragment antigen-binding = Fab) bestehen aus den variablen Regionen beider Ketten, die durch die angrenzenden konstanten Regionen zusammengehalten werden. Weitere Antikörperfragmente sind F(ab')₂-Fragmente, die durch proteolytischen Verdau mit Pepsin hergestellt werden können. Durch Genklonierung können auch verkürzte Antikörperfragmente hergestellt werden, die nur aus den variablen Region der schweren (VH) und der leichten Kette (VL) bestehen. Diese werden als Fv-Fragmente (Fragment variable = Fragment des variablen Teils) bezeichnet. Solche Antikörperfragmente werden auch als *singlechain* Fv-Fragment (scFv) bezeichnet. Beispiele von scFv-Antikörpern sind bekannt und beschrieben, siehe z.B. Huston et al., 1988, Proc. Natl. Acad. Sci. USA, 16, 5879ff.

In den vergangenen Jahren wurden verschiedene Strategien entwickelt um multimere scFv-Derivate herzustellen, wie z.B. Dia-, Tri- und Pentabodies. Als "Diabody" bezeichnet ein Fachmann ein bivalentes homodimeres scFv-Derivat. Die Verkürzung des Peptidlinkers im scFv-Moleküle auf 5 - 10 Aminosäuren resultiert in der Bildung von Homodimeren durch Überlagerung von VH/VL-Ketten. Die Diabodies können zusätzlich durch eingeführte Disulfidbrücken stabilisiert werden. Beispiele von Diabodies finden sich in der Literatur, z.B. bei Perisic et al., 1994 (Structure, 2, 1217ff). Als "Minibody" bezeichnet der Fachmann ein bivalentes, homodimeres scFv-Derivat. Es besteht aus einem Fusionsprotein, das die CH3-Region eines Immunglobulins, vorzugsweise IgG, besonders bevorzugt IgG1, als Dimerisierungsregion enthält. Diese verbindet die scFv-Fragmente über eine Hinge-Region, ebenfalls von IgG, und eine Linker-Region. Beispiele solcher Minibodies sind bei Hu et al., 1996, Cancer Res., 56, 3055ff beschrieben. Mit "Triabody" bezeichnet der Fachmann ein trivalentes homotrimeres scFv-Derivat (Kortt et al., 1997, Protein Engineering, 10, 423ff). Die direkte Fusion von VH-VL ohne Verwendung einer Linkersequenz führt zur Ausbildung von Trimeren.

Bei den vom Fachmann als Mini-Antikörper bezeichneten Fragmenten, die eine bi-, tri- oder tetravalente Struktur haben, handelt es sich ebenfalls um Derivate von scFv-Fragmenten. Die Multimerisierung wird dabei über di-, tri- oder tetramere "coiled coil"-Strukturen erzielt (Pack, P. et al., 1993, Biotechnology, 11, 1271ff; Lovejoy, B. et al., 1993, Science, 259, 1288ff; Pack, P. et al., 1995, J. Mol. Biol., 246, 28ff).

Die entsprechenden Wirkstoffe können zwischen 0,01 bis 80% (w/w) des Gesamtgewichts der Pulver ausmachen. Besonders vorteilhaft sind Pulver, die als Wirkstoff ein Peptid oder Protein oder eine Kombination aus Peptid/Peptid, Peptid/Protein oder Protein/Protein enthalten. Enthalten die erfindungsgemäßen Pulver Wachstumsfaktoren, beispielsweise Cytokine, so beträgt der Anteil normalerweise zwischen 0,1 bis 10% (w/w), vorzugsweise zwischen 0,2 bis 5% (w/w) am Gesamtgewicht des Pulvers. Demnach sind Pulver bevorzugt deren Anteil an Cytokinen 0,2, 0,3, 0,4 ... 0,8, 0,9 etc.; 1, 2, 3, ... etc; 4,1, 4,2, 4,3, ... 4,8 ,4,9 etc.; 4,91, 4,92, 4,93, ... 4,98, 4,99% (w/w) beträgt. Handelt es sich bei dem pharmazeutischen Wirkstoff um Antikörper oder ein Derivat hiervon (bevorzugte Ausführungsform), so beträgt der Wirkstoffanteil zwischen 0,1 und 80% (w/w), vorzugsweise zwischen 1 und 80% (w/w), besonders bevorzugt zwischen 10 und 80% (w/w), weiter bevorzugt zwischen 30 und 80% (w/w), noch weiter bevorzugt zwischen 60 und 80% (w/w), beispielsweise 0,1, 0,2, 0,3, 0,33, ... 0,66, 0,7, 0,8, 0,9 etc.; 1, 2, 3, ... 8 ,9, 10 etc.; 11, 12, 13, ... 18, 19, 20 etc.; 21, 22, 23, ... 28 ,29, 30 etc.; 31, 32, 33, ... 38, 39, 40 etc.; 41, 42, 43, ... 48, 49, 50 etc. 51, 52, 53, ... 58, 59, 60 etc.; 61, 62, 63, ... 68, 69, 70 etc.; 71, 72, 73, ... 78 ,79, etc; 79,1, 79,2, 79,3, ... 79,8, 79,9 etc.; 79,91, 79,92, 79,93, ... 79,98, 79,99, 80% (w/w). am Gesamtgewicht des Pulvers.

Besonders vorteilhaft und erfindungsgemäß sind Pulver mit einem Verhältnis von Matrixbildner zu Peptid/Protein von 21/79, 22/78, 23/77, 24/76, 25/75, 26/74, 27/73, 28/72, 29/71, 30/70, 31/69, 32/68, 33/67, 34/66, 35/65, 36/64, 37/63, 38/62, 39/61, 40/60, 41/59, 42/58, 43/57, 44/56, 45/55, 46/54, 47/53, 48/52, 49/51, 50/50, 51/49, 52/48, 53/47, 54/46, 55/45, 56/44, 57/43, 58/42, 59/41, 60/40, wobei sich die Angaben auf die absoluten Mengen im Pulver beziehen. Enthält das entsprechende Pulver einen oder weitere zusätzliche Hilfsstoffe, beispielsweise die oben beschriebenen Aminosäuren und Di- oder Tri-Peptide, so kann entweder der Anteil an Matrixbildner, der Anteil an pharmazeutischen Wirkstoff, oder beide Anteile entsprechend reduziert werden, wobei der Anteil an Matrixbildner einen der Werte zwischen 21 und 60% (w/w), vorzugsweise zwischen 25 und 50% (w/w), noch weiter bevorzugt zwischen 30 und 40% (w/w) aufweist.

Als Matrixbildner kommen insbesondere die in dieser Anmeldung erwähnten in Betracht. Besonders vorteilhaft sind Pulver die **(a)** als Wirkstoff eines der oben aufgeführten Peptide/Proteine, vorzugsweise einen Antikörper oder ein Derivat hiervon, u n d (b) ein Zucker, Polyol und/oder Polymer, vorzugsweise eine Polyol, besonders bevorzugt Mannitol, in den oben angegebenen Mengenverhältnissen enthalten, wobei sich die Angaben jeweils auf die absolute Menge im Pulver beziehen.

Als besonders vorteilhaft und dem entsprechend auch erfindungsgemäß haben sich Pulver gezeigt, die **(a)** als Wirkstoff einen Antikörper enthalten u n d **(b)** als Matrixbildner ein Polyol, insbesondere Mannitol in einer Konzentration von über 20% (w/w), vorzugsweise von 25-60% (w/w), besonders bevorzugt von 25-50% (w/w), noch weiter bevorzugt von 30-40% (w/w) enthalten, und **(c)** der Restwassergehalt des besagten Pulvers unterhalb von 1,2% (w/w), vorzugsweise unterhalb von 1,0% (w/w) liegt.

Nach einer weiteren Ausführungsform betrifft die vorliegende Erfindung auch Pulver die (a) als Wirkstoff einen Antikörper enthalten (b) als Matrixbildner ein Polyol, insbesondere Mannitol in einer Konzentration von über 20% (w/w), vorzugsweise von 25-60% (w/w), besonders bevorzugt von 25-50% (w/w), noch weiter bevorzugt von 30-40% (w/w) enthalten, und (c) ein Tri-Peptid, mit zumindest einem Isoleucinrest wie zum Beispiel Tri-Isoleucin in einer Konzentration von 1-19.99% (w/w) enthalten, u n d (d) der Restwassergehalt des besagten Pulvers unterhalb von 1,2% (w/w), vorzugsweise unterhalb von 1,0% (w/w) liegt.

Mit der entsprechenden Pulverformulierung war es in überraschender Weise gelungen Polyole, insbesondere Mannitol in höheren Konzentrationen als Matrixbildner bei der Herstellung von Pulvern, die Antikörper als pharmazeutischen Wirkstoff enthalten, einzusetzen, und so die vorteilhaften Eigenschaften der Polyole, insbesondere von Mannitol, zu nutzen. Dies war um so überraschender, da Proteine normalerweise durch Übertrocknung, also bei Verringerung des Restfeuchte auf weniger als 1% (w/w), destabilisiert werden (Breen et al., 2001, Pharm Res., 18(9), *supra*).

### Herstellung der erfindungsgemäßen Pulver

Durch Gefriertrocknung können Pulver hergestellt werden, deren Restfeuchten < 1 % (w/w) sind, ähnliches gilt auch für die Vakuumtrocknung. Für die Herstellung von Formulierungen zur inhalativen Applikation können diese Pulver nicht verwendet werden, da diese Verfahren nicht zu inhalierbaren Partikeln führen. Eine Alternative bietet die Sprühtrocknung, die es erlaubt hinreichend kleine Partikel herzustellen. Die erzielten Restfeuchten liegen allerdings zumeist deutlich höher als bei der Gefriertrocknung. Dies trifft insbesondere auf sprühgetrocknete Pulver zu, die temperaturlabile Peptide/Proteine als pharmazeutischen Wirkstoff enthalten. Insbesondere Antikörper oder deren Derivate lassen sich normalerweise nur bei relativ niedrigen Einströmtemperaturen (*inlet*-Temperaur < 150°C) sprühtrocknen.

Im Rahmen der vorliegenden Erfindung wurde überraschenderweise gefunden, dass eine effiziente Nachtrocknung sprühgetrockneter Pulver, die als Matrixbildner Zucker oder Polyole, vorzugsweise Mannitol, im Bereich von 20-60% (w/w) enthalten, besonders lagerstabil sind, insbesondere bei Temperaturen von größer 20°C, und sich durch eine hohe Dispergierbarkeit auszeichnen. Dies trifft im Besonderen auf die inhalierbaren sprühgetrockneten Pulver zu. Durch eine spezielle Vakuumtrocknung wurden die Restfeuchten der sprühgetrockneten Pulver auf unter 1,2% (w/w) gesenkt, vorzugsweise auf unter 1% (w/w) was zu einer signifikant verbesserten Proteinstabilität und Dispergierbarkeit führt. Dabei war insbesondere die verbesserte Stabilität der Proteine durch Vakuumtrocknung bei 25-60°C, vorzugsweise bei Temperaturen von über 30°C (32°C), mit einem Vakuum von 0,01-1 mbar, vorzugsweise von 0,1 mbar, über 24 Stunden überraschend und nicht vorhersehbar. Denn aus der Literatur ist bekannt, dass eine Übertrocknung von Proteinen zu einer Abnahme der Stabilität führt. Übertrocknung meint in diesem Zusammenhang eine Reduktion der Restfeuchte auf weniger als 1% (vgl. auch Breen et al., 2001, *supra*). Die durch die vorliegende Erfindung bereitgestellten Pulver mit besonderer Stabilität wiesen zumeist Restfeuchten von weniger als 1% (w/w) auf.

Die vorliegende Erfindung stellt somit ein Verfahren zur Herstellung eines der oben näher beschriebenen sprühgetrockneten Pulvers zur Verfügung *dadurch gekennzeichnet,* dass eine Sprühlösung enthaltend zumindest einen pharmazeutischen Wirkstoff und einen Matrixbildner (a) unterhalb einer Temperatur von 200/120°C vorzugsweise 150/70°C versprüht wird, u n d (b) das resultierende Pulver anschließend unter Vakuum bei einer Temperatur von 25-60°C nachgetrocknet wird.

Sofern neben dem pharmazeutischen Wirkstoff und Matrixbildner weitere Hilfsstoffe enthalten sind, wie zum Beispiel Aminosäuren (Isoleucin) oder Peptide, (Isoleucinhaltige Di- oder Tri-Peptide), so werden diese natürlich ebenfalls der Sprühlösung zugesetzt. Vorzugsweise werden die oben beschriebenen Kombinationen aus Matrixbildner und zusätzlichen Hilfsstoffe zur Herstellung einer Sprühlösung verwendet. Hierzu wird der therapeutische Wirkstoff, vorzugsweise ein biologisches Makromolekül in Form eines Peptids oder Proteins, in einer wässrigen Lösung gelöst, abhängig von den Löslichkeitsbedingungen des jeweiligen Wirkstoffs. Zumeist werden gepufferte Lösungen mit einem pH von 3-11, vorzugsweise von 4-9 verwendet. Bei der Herstellung von inhalierbaren Pulvern ist eine wässrige Lösung mit einem pH von 5,5-7,8 besonders vorteilhaft. Um eine hinreichende Löslichkeit zu gewähren, sollte der pH-Wert der Lösung unterhalb des pI-Werts des Peptids/Proteins liegen. Die wässrige Lösung kann optional zusätzliche wasserlösliche organische Lösungsmittel enthalten, wie z.B. Aceton, Alkohole oder der gleichen. Besonders geeignet sind niedere Alkohole wie z.B. Methanol, Ethanol, Propanol, (n- oder iso-Propanol) oder der gleichen. Solche gemischten Lösungsmittelsysteme enthalten normalerweise zwischen 0,1-80% (v/v), vorzugsweise zwischen 10-40% (v/v), und besonders bevorzugt zwischen 10-20% (v/v) eines wasserlöslichen organischen Lösungsmittels. Der Feststoffanteil in der zu versprühenden Lösung beträgt üblicherweise zwischen 0,01-20% (w/v), vorzugsweise zwischen 0,05-10% (w/v), besonders bevorzugt zwischen 0.1-2% (w/v). In Rahmen der vorliegenden Erfindung wurden sprühgetrocknete Pulver ausgehend von einer Lösung mit einem Feststoffanteil von 10% (w/v) hergestellt Matrixbildner und weitere Hilfsstoffe, sofern vorhanden, werden zusammen mit dem Wirkstoff oder separat gelöst und versprüht.

Die Versprühung erfolgt in konventionellen Sprühtrocknern, beispielsweise in Geräten der Fa. Niro A/S (Soeborg, DK), Büchi Labortechnik GmbH (Flawil, CH) oder der gleichen. Die optimalen Bedingungen für die Sprühtrocknung hängen jeweils von der entsprechenden Formulierung ab und sind experimentell zu bestimmen. Als Gas wir typischerweise Luft verwendet, geeignet sind aber auch inerte Gase wie Stickstoff oder Argon. Darüber hinaus bestimmt sich die Sprühtrocknungstemperatur, gemeint sind Einström- (*inlet*-) und Ausströmtemperatur (*outlet*-temperature), nach der Temperatursensitivität des verwendeten Wirkstoffs, jeweils in Abhängigkeit der verwendeten Stabilisatoren. Üblich ist eine *inlet*-Temperatur von 50-200°C während die *outlet*-Temperatur zumeist 30-150°C beträgt. Im Rahmen der vorliegenden Erfindung wurde mit einer *inlet*-Temperatur von ungefähr 105°C und einer *outlet*-Temperatur von 50-55°C gearbeitet. Möglich sind allerdings auch etwas höhere Temperaturen, beispielsweise eine *inlet*-Temperatur von 120-200°C, vorzugsweise 90-130°C und eine *outlet*-Temperatur von 70-120°C, vorzugsweise 50-80°C, je nach Stabilisatoranteil. Die Versprühung erfolgt in der Regel bei einem Druck von ungefähr 20-150 psi, vorzugsweise bei ungefähr 30- oder 40-100 psi, beispielsweise bei ungefähr 30, 40, 50, 60, 70, 80, 90 oder 100 psi.

Zur Nachtrocknung eignet sich besonders die Trocknung unter Vakuum. Diese erlaubt eine schonende Trocknung bei Temperaturen von 15-60°C, vorzugsweise zwischen 20-40°C. Vorliegend wurde eine Temperatur von mehr als 30°C verwendet. Die jeweils optimale Temperatur hängt von der jeweiligen Zusammensetzung des zu trocknenden Pulvers und den weiteren Trocknungsbedingungen ab (z.B. Trocknungsdauer). Zur Trocknung eignet sich ein Vakuum von 0,01-10 mbar, vorzugsweise von 0,02-5 mbar, weiter bevorzugt von 0,02-1 mbar. Als besonders vorteilhaft hat sich die Trocknung bei 0,05-0,5 mbar erwiesen. Die Trocknungsdauer lag zumeist bei weniger :als 30 Std., vorzugsweise bei ungefähr 24 Std. Vorstellbar sind auch Trocknungszeiten von weniger als 24 Std, beispielsweise 18, 15 oder 12 Std. Vorstellbar ist aber auch eine Trocknung bei niedrigeren Temperaturen (ca. 20°C oder darunter). Dies setzt allerdings zumeist ein größeres Vakuum (= niedrigere Drücke) voraus. Beim Nachtrocknen von Pulvern mit einem Gewichtsanteil von ≥ 30% an Polyolen, insbesondere an Mannitol, hat sich eine Trocknungstemperatur von ungefähr 25-35°C, ein Vakuum von 0,05-0,2 mbar und die Trocknungsdauer auf 18-30 Std. als vorteilhaft erwiesen. Diese Bedingungen führten zu besonders lagerstabilen amorphen Pulvern mit einem geringen Anteil an Aggregaten, vorzugsweise von < 3,5% auch bei extremen Lagerbindungen von 15 Wochen bei 40°C und 75% rF.

Demnach betrifft die vorliegende Erfindung in einer weiteren Ausführungsform ein Verfahren zur Herstellung eines sprühgetrockneten Pulvers *dadurch gekennzeichnet,* dass eine Sprühlösung enthaltend zumindest einen pharmazeutischen Wirkstoff und ein Polyol als Matrixbildner, vorzugsweise Mannitol, in einer Konzentration von mehr als 20 aber weniger als 60% (w/w) sowie als einen oder mehrere zusätzliche Hilfsstoffe Tripeptide der allgemeinen Formel Ile-X-X, X-Ile-X,X-X-Ile **(a)** unterhalb von einer Temperatur von 105/60°C versprüht wird; und **(b)** das resultierende Pulver anschließend unter Vakuum bei einem Druck von ungefähr 0,05-0,2 mbar, einer Temperatur von 25-60°C, vorzugsweise bei 25-35°C für ungefähr 18-30 Std., vorzugsweise ungefähr 24 Std. nachgetrocknet wird. Nach einer weiteren Ausführungsform werden Matrixbildner und pharmazeutischer Wirkstoff in separaten Sprühlösungen gelöst aber gemeinsam versprüht. Zumindest eine der zu versprühende Lösungen kann ferner weitere Hilfsstoffe, wie zum Beispiel Aminosäuren, vorzugsweise Isoleucin, Peptide in Form von Tri- oder DiPeptiden, vorzugsweise mit einem Isoleucinrest, beispielsweise Tri-Isoleucin, enthalten. Die Sprühlösungen sind insgesamt so beschaffen, dass durch Versprühung Pulver mit den in dieser Patentschrift beschriebenen Zusammensetzung entstehen.

### Beschaffenheit der erfindungsgemäßen Pulver

Die Pulver der vorliegenden Erfindung zeichnen sich durch ihre vorteilhaften Eigenschaften aus, die in einer konstant guten Dispergierbarkeit, auch nach längeren Lagerungszeiten, in einer sehr guten Langzeitstabilität, und einer guten Inhalierbarkeit bis in den unteren Lungentrakt bestehen. Letzteres wird u.a. durch die mittlere Teilchengröße, den mittleren aerodynamischen Teilchendurchmesser (MMAD) und den Anteil der sog. "*Fine Particle Fraction*" (FPF) bestimmt.

Die Partikel der vorliegenden verfügen vorzugsweise über eine mittlere Teilchengröße von weniger als 20 µm, vorzugsweise von weniger als 10 µm. Nach einer besonders bevorzugten Ausführungsform verfügen die erfindungsgemäßen Partikel über einen mittlere Teilchengröße von weniger als 7,5 µm, vorzugsweise von weniger als 5 µm. Besonders bevorzugt sind Partikel mit einem mittlere Teilchengröße von weniger als 4 µm und weiter bevorzugt von weniger als 3,5 µm. Im allgemeinen weisen die Partikel der vorliegenden Erfindung einen mittleren Teilchendurchmesser von 0,1-5 µm, vorzugsweise von 0,2-4 µm auf. In einer weiteren Ausführungsform enthalten die entsprechenden Partikel nicht-respirierbare Teilchen, z.B. Laktose, mit einer Teilchengröße von zumindest 40 µm, vorzugsweise zwischen 40 und 200 µm.

Neben der mittleren Teilchengröße hängt die Inhalierbarkeit im wesentlichen von dem mittleren aerodynamischen Teilchendurchmesser (MMAD) ab. Die erfindungsgemäßen Partikel verfügen über einen MMAD von kleiner 10 µm vorzugsweise von weniger als 7,5 µm auf. Besonders vorteilhaft sind Partikel mit einem MMAD von kleiner 5 µm, vorzugsweise von kleiner 4 µm, noch weiter bevorzugt von kleiner 3,5 µm. Nach einer weiter bevorzugten Ausführungsform der vorliegenden Erfindung verfügen die Partikel über einen MMAD von kleiner 3 µm. Im allgemeinen weisen die Partikel einen MMAD von 0,5-10 µm, vorzugsweise von 0,5-7,5 µm, vorzugsweise von 1-5 µm auf. Die in den Beispielen beschriebenen Partikel weisen einen entsprechend vorteilhaften mittleren aerodynamischen Teilchendurchmesser auf, die durch die Kombination aus optimaler Sprühtrocknungsbedingungen und der erfindungsgemäßen Wahl und Konzentration des Matrixbildners sowie weitere Hilfsstoffe, sofern vorhanden, definiert werden.

Die erfindungsgemäßen Partikel sind ferner durch ihre spezifische Dichte definiert. Die erfindungsgemäßen Partikel verfügen im allgemeinen über eine bulk-Dichte von 0,1-10g/cm³, vorzugsweise von 0,1-2g/cm³. Nach einer besonders bevorzugten Ausführungsform verfügen die erfindungsgemäßen Partikel über eine bulk-Dichte von 1,1-1,5 g/cm³.

Die erfindungsgemäßen Partikel zeichnen sich insbesondere durch ihre sehr geringe Restfeuchte im Pulver auf, die die überraschenden erfindungsgemäßen Eigenschaften der Partikel begründen. Die erfindungsgemäßen Partikel weisen nach Sprühtrocknung im allgemeinen einen Restwassergehalt von bis zu 10% (w/w) auf, vorzugsweise von 2-9% (w/w). Nach anschließender Nachtrocknung, vorzugsweise unter Vakuum, weisen die erfindungsgemäßen eine Restwassergehalt von weniger als 1,2% (w/w), vorzugsweise von weniger als 1,1% (w/w). Besonders bevorzugt sind Partikel mit einem Restwassergehalt von weniger als 1,0% (w/w), vorzugsweise von weniger als 0,9% (w/w), noch mehr bevorzugt von weniger als 0,8% (w/w). Im allgemeinen weisen die erfindungsgemäßen Pulver nach Nachtrocknung einen Restwassergehalt von 0,5-1,2% (w/w), vorzugsweise von 0,6-1% (w/w), noch weiter bevorzugt von 0,7-0,9% (w/w) auf. Die in den erfindungsgemäßen Pulver enthaltenen Partikel sind vorwiegend hygroskopisch.

Der geringe Restwassergehalt lässt sich durch eine Kombination von Sprühtrocknung und Nachtrocknung unter Vakuum einstellen.

Die erfindungsgemäßen Partikel zeichnen sich durch ihre hohe und konstante Dispergierbarkeit aus. Die Sprühtrocknungsbedingungen sowie die verwendeten Pulverformulierungen (z.B. Wahl und Konzentration des Matrixbildners sowie weiterer Hilfsstoffe sofern vorhanden) bestimmen im wesentlichen die Dispergierbarkeit. Die erfindungemäßen Partikel zeichnen sich dadurch aus, dass der Anteil der ausgebrachten Menge größer 30%, vorzugsweise größer 40%, und in bevorzugter Weise größer 50% ist. In einer weiter bevorzugten Ausführungsform beträgt der Anteil an ausgebrachter Menge mehr als 60%, vorzugsweise mehr als 70%, besonders bevorzugt mehr als 75%, noch weiter bevorzugt bei mehr als 80%. Die in den Beispielen beschriebenen Pulverzubereitungen verfügten über einen Anteil an ausgebrachter Menge zwischen 60-90%. Als besonders dispergierbar erwiesen sich hierbei die Pulver mit einem Mannitolgehalt von 30 bis 40% (w/w) und einem Restwassergehalt von weniger als 1,2% (w/w), vorzugsweise von weniger als 1% (w/w), noch weiter bevorzugt von weniger als 0,9 % (w/w) oder sogar 0,8% (w/w). Der Anteil an ausgebrachter Menge, gemessen mit einem Handihaler®, betrug über 80%.

Ein weiteres Maß für die gute Performance der Partikel ist der Anteil der "*Fine Partikel Fraction*" in den Pulverzubereitungen. Die erfindungsgemäßen Pulverzubereitungen weisen nach einer weiteren erfindungsgemäßen Ausführungsform einen Anteil an FPF_{(7,5)}, meint einen Anteil an Teilchen mit einen MMAD von kleiner 7,5 µm, von zumindest 35% vorzugsweise von zumindest 40%, weiter bevorzugt von zumindest 45%, noch weiter bevorzugt von zumindest, 50%, wie z.B. von 55%, 60%, 65%, etc., auf. Nach einer weiteren bevorzugten Ausführungsform der vorliegenden Erfindung weisen die Pulverzubereitungen mit einem Anteil an FPF_{(3,5)}, meint einen Anteil an Teilchen mit einen MMAD von 3,5-0,5 µm, von zumindest 30%, vorzugsweise von zumindest 35%, beispielsweise von zumindest 40, 45, 50, 55, 60% auf. Eine entsprechende Größenverteilung eignet sich besonders gut für die Inhalation bis in den tiefen Lungentrakt.

Die durch diese Erfindung bereitgestellten Pulverzubereitungen zeichnen sich ferner durch eine sehr gute Langzeitstabilität, meint durch eine gleich bleibende Pulverperformance über mehrere Wochen bei extremen Lagerbedingungen, aus. Hiezu tragen die überraschend gute physikalische und chemische Stabilität der Pulver bei. Der Anteil, des im Pulver in aggregierter Form vorliegende pharmazeutischen Wirkstoffs, beträgt für die erfindungsgemäßen Partikel weniger als 3,5%, vorzugsweise weniger als 3,2% in Bezug auf die im Pulver enthaltene Gesamtwirkstoffmenge. Nach einer bevorzugten Ausführungsform beträgt der Anteil an aggregiertem Wirkstoff weniger als 3,0%, vorzugsweise weniger als 2,8%, beispielsweise weniger als 2,7, 2,6, 2,5, 2,4, 2,3, 2,2, 2,1, 2,0, 1,9, 1,8%, etc, wiederum bezogen auf die im Pulver vorhandene Gesamtwirkstoffmenge. Dies schließt eine Lagerung für 4 teilweise auch für 15 Wochen bei 30 bzw. 40°C und einer relativen Feuchte von 60 bzw. 75% mit ein. Demnach beträgt der Anteil an freiem monomeren Wirkstoff typischerweise mehr als 95%, vorzugsweise mehr als 96,5%, weiter bevorzugt mehr als 96,8%, beispielsweise 97,0, 97,1, 97,2, 97,3, 97,4, 97,5, 97,6, 97,7, 97,8, 97,9, 98,0, 98,1, 98,2% etc, wiederum bezogen auf die Gesamtwirkstoffmenge im Pulver. Die in den Beispielen dargestellten Pulver, insbesondere die mit einem Polyolanteil von 20-40% (w/w), insbesondere von 30-40% (w/w) wiesen nach 15 wöchiger Lagerung bei einer relativen Feuchte von 75% einen Anteil an nicht aggregiertem Antikörper von größer 96,5% auf (Anteil an Aggregaten lag bei ca. 3,2-3,3%).

### Verabreichung der erfindungsgemäßen Pulver

Grundsätzlich lassen sich die erfindungsgemäßen Pulverzubereitungen direkt als trockenes Pulver über sog. Trockenpulver-Inhalatoren, oder aber nach Rekonstitution in Form von Aerosolen über sog. Vernebler applizieren. Die erfindungsgemäßen Inhalationspulver können dabei mittels aus dem Stand der Technik bekannten Inhalatoren appliziert werden.

Erfindungsgemäße Inhalationspulver können beispielsweise mittels Inhalatoren appliziert werden, die eine einzelne Dosis aus einem Vorrat mittels einer Messkammer, wie er in der US 4,570,630A beschrieben wird, oder über andere apparative Vorrichtungen, wie sie in der DE 36 25 685 A beschrieben werden, appliziert werden. Vorzugsweise werden die erfindungsgemäßen Inhalationspulver in Kapseln abgefüllt (zu sogenannten Inhaletten), die in Inhalatoren, wie beispielsweise in der WO 94/28958 beschrieben, zur Anwendung gelangen.

Weitere Beispiele für geeignete Inhalatoren finden sich u.a. in US 5,458,135; US 5,785,049 oder WO 01/00263. Weitere geeignete Inhalatoren sind aus der WO 97/41031; US 3,906,950 und US 4,013,075 bekannt. Weitere Dispersionsinhalatoren für trockene Pulverzubereitungen sind in EP 129 985; EP 472 598; EP 467 172 und US 5,522,385 beschrieben.

Die erfindungsgemäßen Inhalationspulver können beispielsweise mittels des unter dem Namen Turbuhaler® (AstraZeneca LP) bekannten Inhalators beziehungsweise mit Inhalatoren wie sie beispielsweise in der EP 237 507 A offenbart werden, appliziert werden. Andere geeignete Inhalatoren sind der Rotahaler® oder der Discus® (beide von GlaxoSmithKline Corp.), der Spiros^{™} Inhaler (Dura Pharmaceuticals) und der Spinhaler® (Fiscon).

Ein zur Anwendung der erfindungsgemäßen Arzneimittelkombination in Inhaletten besonders bevorzugter Inhalator ist der Figur 5 zu entnehmen. Dieser Inhalator (Handihaler) für die Inhalation pulverförmiger Arzneimittel aus Kapseln ist gekennzeichnet durch ein Gehäuse 1, enthaltend zwei Fenster 2, ein Deck 3, in dem sich Lufteinlassöffnungen befinden und welches mit einem über ein Siebgehäuse 4 befestigten Sieb 5 versehen ist, eine mit Deck 3 verbundene Inhalationskammer 6, an der ein mit zwei geschliffenen Nadeln 7 versehener, gegen eine Feder 8 beweglicher Drücker 9 vorgesehen ist, sowie ein über eine Achse 10 klappbar mit dem Gehäuse 1, dem Deck 3 und einer Kappe 11 verbundenes Mundstück 12, sowie Luftdurchlasslöcher 13 zur Einstellung des Strömungswiderstandes.

Sollen die erfindungsgemäßen Inhalationspulver im Sinne der vorstehend gennannten bevorzugten Anwendung in Kapseln (Inhaletten) abgefüllt werden, bieten sich Füllmengen von 1 bis 30mg pro Kapsel an.

Die erfindungsgemäßen Pulver können ferner als treibgashaltige Inhaltionsaerosole appliziert werden. Hierzu werden die erfindungsgemäßen Pulver in einer wässrigen Lösung rekonstituiert. Geeignete Lösungen sind im Stand der Technik bekannt. Vorteilhaft ist beispielsweise die Rekonstitution in physiologischen Lösungen mit einem pH von 3-11, vorzugsweise von 4-9. Besonders vorteilhaft ist die Rekonstitution in einer wässrigen Lösung mit einem pH von 5,5-7,8. Die Lösung zur Rekonstitution der erfindungsgemäßen Pulver kann ferner weitere Hilfsstoffe in Form von Stabilisatoren, Emulgatoren oberflächen aktiven Substanzen, wasserlöslichen organischen Lösungsmitteln enthalten. Entsprechende Substanzen sind dem Fachmann bekannt, und beispielhaft in (Bauer, Lehrbuch der Pharmazeutischen Technologie, Wissenschaftl. Verlagsgesellschaft mbH, Stuttgart, 178-184; Adler, 1998, Journal of Pharmaceutical Sciences, 88(2), 199-208) beschrieben. Entsprechende Inhalationsaerosole, die durch Rekonstitution der efindungsgemäßen Pulver hergestellt werden, sind ebenfalls Gegenstand der vorliegenden Erfindung.

Die zur Herstellung der erfindungsgemäßen Inhalationsaerosole einsetzbaren Treibgase sind ebenfalls aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie bevorzugt chlorierten und fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind halogenierte Alkanderivate ausgewählt aus TG11, TG12, TG134a (1,1,1,2-Tetrafluorethan), TG227 (1,1,1,2,3,3,3-Heptafluorpropan) und Mischungen derselben, wobei die Treibgase TG134a, TG227 und Gemische derselben bevorzugt sind.

Die erfindungsgemäßen treibgashaltigen Inhalationsaerosole können bis zu 5% (w/w) an Wirkstoff enthalten. Erfindungsgemäße Aerosole enthalten beispielsweise 0,002-5% (w/w), 0,01-3% (w/w), 0,015-2% (w/w), 0,1-2% (w/w), 0,5-2% (w/w) oder 0,5-1% (w/w) an dem pharmazeutischen Wirkstoff. Inhaltationsaerosole mit einer entsprechenden Wirkstoffkonzentration lassen sich durch gezielte Rekonstitution der erfindungsgemäßen Pulver in einer entsprechenden Menge an Lösungsmittel einstellen.

Die vorstehend genannten erfindungsgemäßen treibgashaltigen Inhalationaerosole können mittels im Stand der Technik bekannten Inhalatoren (MDIs = metered dose inhalers) appliziert werden. Beispielhaft sei hier auf den Ventolin® (Ventolin Pharmacy) oder die in US 5,32,094 oder US 5,672,581 beschriebenen Inhalatoren verwiesen. Dementsprechend betrifft ein weiterer Aspekt der vorliegenden Erfindung Arzneimittel in Form von wie vorstehend beschriebenen treibgashaltigen Aerosolen in Verbindung mit einem oder mehreren zur Verabreichung dieser Aerosole geeigneten Inhalatoren. Ferner betrifft die vorliegende Erfindung Inhalatoren, dadurch gekennzeichnet, dass sie vorstehend beschriebene erfindungsgemäße treibgashaltige Aerosole enthalten.

Die vorliegende Erfindung betrifft ferner Kartuschen, die ausgestattet mit einem geeigneten Ventil in einem geeigneten Inhalator zur Anwendung gelangen können und die eine der vorstehend genannten erfindungsgemäßen treibgashaltigen Inhalationsaerosole enthalten. Geeignete Kartuschen und Verfahren zur Abfüllung dieser Kartuschen mit den erfindungsgemäßen treibgashaltigen Inhaltionsaerosolen sind aus dem Stand der Technik bekannt.

Die erfindungsgemäßen Pulver können ferner in treibgasfreien Inhalationslösungen oder Suspensionen rekonstituiert werden. Entsprechende treibgasfreie Inhalationslösungen enthalten beispielsweise wässrige oder alkoholische, bevorzugt ethanolische, gegebenenfalls ethanolische im Gemisch mit wässrigen Lösungsmitteln. Im Falle wässrig/ethanolischer Lösungsmittelgemische ist der relative Anteil an Ethanol gegenüber Wasser nicht begrenzt, bevorzugt liegt die maximale Grenze jedoch bei bis 70% (v/v), insbesondere bei bis zu 60% (v/v) Ethanol. Die restlichen Volumenprozente werden von Wasser aufgefüllt. Den erfindungsgemäßen treibgasfreien Inhalationslösungen können Co-Solventien und/oder weitere Hilfsstoffe, wie oben beschrieben, zugesetzt werden. Beispielsweise lassen sich Co-Solventien verwenden, die Hydroxylgruppen oder andere polare Gruppen enthalten, wie z.B. Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen neben den oben aufgeführten z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmacksstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien. Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine. Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besondersbevorzugt zwischen 5 und 20 mg/100ml enthalten. Demnach umfasst die vorliegende Erfindung auch treibgasefreie Inhaltationsaerosole, die durch Rekonstituion der erfindungsgemäßen Pulver hergestellt werden.

Zur Applikation der erfindungsgemäßen treibgasfreien Inhaltionslösungen sind besonders solche Inhalatoren geeignet, die eine kleine Menge einer flüssigen Formulierung in der therapeutisch notwendigen Dosierung binnen weniger Sekunden in ein therapeutisch-inhalativ geeignetes Aerosol vernebeln können. Im Rahmen der vorliegenden Erfindung sind solche Vernebler bevorzugt, bei denen bereits eine Menge von weniger als 100 µL, bevorzugt weniger als 50 µL, besonders bevorzugt zwischen 10 und 30 µL Wirkstofflösung mit bevorzugt einem Hub zu einem Aerosol mit einer durchschnittlichen Teilchengröße von weniger als 20 µm, bevorzugt weniger als 10 µm, so vernebelt werden können, so dass der inhalierbare Anteil des Aerosols bereits der therapeutisch wirksamen Menge entspricht.

Eine derartige Vorrichtung zur treibgasfreien Verabreichung einer dosierten Menge eines flüssigen Arzneimittels zur inhalativen Anwendung, wird beispielsweise in der internationalen Patentanmeldung WO 91/14468 als auch in der WO 97/12687 (dort insbesondere in den Figuren 6a und 6b) ausführlich beschrieben. Auf die entsprechenden Figuren 6a und 6b der WO 97/12687 einschließlich der dazugehörigen Beschreibungsteile wird im Rahmen der vorliegenden Erfindung ausdrücklich Bezug genommen. Die dort beschriebenen Vernebler (Devices) sind auch unter der Bezeichnung Respimat® (Boehringer Ingelheim Pharma) bekannt. Aufgrund seiner zylinderähnlichen Form und einer handlichen Größe von weniger als 9 bis 15 cm in der Länge und 2 bis 4 cm in der Breite kann dieses Device jederzeit vom Patienten mitgeführt werden. Der Vernebler versprüht ein definiertes Volumen der Arzneimittelformulierung unter Anwendung hoher Drücke durch kleine Düsen, so dass inhalierbare Aerosole entstehen.

Im wesentlichen besteht der bevorzugte Zerstäuber aus einem Gehäuseoberteil, einem Pumpengehäuse, einer Düse, einem Sperrspannwerk, einem Federgehäuse, einer Feder und einem Vorratsbehälter, gekennzeichnet durch
- ein Pumpengehäuse, das im Gehäuseoberteil befestigt ist, und das an seinem einen Ende einen Düsenkörper mit der Düse bzw. Düsenanordnung trägt,
- einen Hohlkolben mit Ventilkörper,
- einen Abtriebsflansch, in dem der Hohlkolben befestigt ist, und der sich im Gehäuseoberteil befindet,
- ein Sperrspannwerk, das sich im Gehäuseoberteil befindet,
- ein Federgehäuse mit der darin befindlichen Feder, das am Gehäuseoberteil mittels eines Drehlagers drehbar gelagert ist,
- ein Gehäuseunterteil, das auf das Federgehäuse in axialer Richtung aufgesteckt ist.

Der Hohlkolben mit Ventilkörper entspricht einer in der WO 97/12687 offenbarten Vorrichtung. Er ragt teilweise in den Zylinder des Pumpengehäuses hinein und ist im Zylinder axial verschiebbar angeordnet. Insbesondere wird im Rahmen der vorliegenden Erfindung auf die Figuren 1-4 - insbesondere auf Figur 3 - und die dazugehörigen Beschreibungsteile Bezug genommen. Der Hohlkolben mit Ventilkörper übt auf seiner Hochdruckseite zum Zeitpunkt des Auslösens der Feder einen Druck von 5 bis 60 Mpa (etwa 50 bis 600 bar), bevorzugt 10 bis 60 Mpa (etwa 100 bis 600 bar) auf das Fluid, die abgemessene Wirkstofflösung aus. Dabei werden Volumina von 10 bis 50 Mikroliter bevorzugt, besonders bevorzugt sind Volumina von 10 bis 20 Mikroliter, ganz besonders bevorzugt ist ein Volumen von 15 Mikroliter pro Hub.

Der Ventilkörper ist bevorzugt an dem Ende des Hohlkolbens angebracht, das dem Düsenkörper zugewandt ist.

Die Düse im Düsenkörper ist bevorzugt mikrostrukturiert, d.h. durch Mikrotechnik hergestellt. Mikrostrukturierte Düsenkörper sind beispielsweise in der WO 94/07607 offenbart; auf diese Schrift wird hiermit inhaltlich Bezug genommen, insbesondere auf die dort offenbarte Figur 1 und deren Beschreibung. Der Düsenkörper besteht z.B. aus zwei fest miteinander verbundenen Platten aus Glas und/oder Silizium, von denen wenigstens eine Platte einen oder mehrere mikrostrukturierte Kanäle aufweist, die die Düseneinlassseite mit der Düsenauslassseite verbinden. Auf der Düsenauslassseite ist mindestens eine runde oder nicht-runde Öffnung von 2-10 µm Tiefe und 5-15 µm Breite, wobei die Tiefe bevorzugt bei 4, 5 bis 6,5 Mikrometern und die Länge bei 7-9 Mikrometern beträgt. Im Fall von mehreren Düsenöffnungen, bevorzugt sind zwei, können die Strahlrichtungen der Düsen im Düsenkörper parallel zueinander verlaufen oder sie sind in Richtung Düsenöffnung gegeneinander geneigt. Bei einem Düsenkörper mit mindestens zwei Düsenöffnungen auf der Auslassseite können die Strahlrichtungen mit einem Winkel von 20-160 Grad gegeneinander geneigt sein, bevorzugt wird ein Winkel von 60-150 Grad, insbesondere bevorzugt 80-100°. Die Düsenöffnungen sind bevorzugt in einer Entfernung von 10-200 µm angeordnet, stärker bevorzugt in einer Entfernung von 10-100 µm, besonders bevorzugt 30-70 µm. Am stärksten bevorzugt sind 50 µm.

Die Strahlrichtungen treffen sich dementsprechend in der Umgebung der Düsenöffnungen.

Die flüssige Arzneimittelzubereitung trifft mit einem Eingangsdruck von bis zu 600 bar, bevorzugt 200 bis 300 bar auf den Düsenkörper und wird über die Düsenöffnungen in ein inhalierbares Aerosol zerstäubt. Die bevorzugten Teilchen- bzw. Tröpfchengrößen des Aerosols liegen bei bis zu 20 µm, bevorzugt bei 3-10 µm.

Das Sperrspannwerk enthält eine Feder, bevorzugt eine zylindrische schraubenförmige Druckfeder, als Speicher für die mechanische Energie. Die Feder wirkt auf den Abtriebsflansch als Sprungstück, dessen Bewegung durch die Position eines Sperrglieds bestimmt wird. Der Weg des Abtriebsflansches wird durch einen oberen und einen unteren Anschlag präzise begrenzt. Die Feder wird bevorzugt über ein kraftübersetzendes Getriebe, z.B. ein Schraubschubgetriebe, durch ein äußeres Drehmoment gespannt, das beim Drehen des Gehäuseoberteils gegen das Federgehäuse im Gehäuseunterteil erzeugt wird. In diesem Fall enthalten das Gehäuseoberteil und der Abtriebsflansch ein ein- oder mehrgängiges Keilgetriebe.

Das Sperrglied mit einrückenden Sperrflächen ist ringförmig um den Abtriebsflansch angeordnet. Es besteht z.B. aus einem in sich radial elastisch verformbaren Ring aus Kunststoff oder aus Metall. Der Ring ist in einer Ebene senkrecht zur Zerstäuberachse angeordnet. Nach dem Spannen der Feder schieben sich die Sperrflächen des Sperrgliedes in den Weg des Abtriebsflansches und verhindern das Entspannen der Feder. Das Sperrglied wird mittels einer Taste ausgelöst. Die Auslösetaste ist mit dem Sperrglied verbunden oder gekoppelt. Zum Auslösen des Sperrspannwerkes wird die Auslösetaste parallel zur Ringebene, und zwar bevorzugt in den Zerstäuber hinein, verschoben; dabei wird der verformbare Ring in der Ringebene verformt. Konstruktive Details des Sperrspannwerkes sind in der WO 97/20590 beschrieben.

Das Gehäuseunterteil wird in axialer Richtung über das Federgehäuse geschoben und verdeckt die Lagerung, den Antrieb der Spindel und den Vorratsbehälter für das Fluid.

Beim Betätigen des Zerstäubers wird das Gehäuseobereil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganzzahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgezogen, wodurch eine Teilmenge des Fluids aus dem Vorratsbehälter in den Hochdruckraum vor der Düse eingesaugt wird.

In den Zerstäuber können gegebenenfalls nacheinander mehrere das zu zerstäubende Fluid enthaltende austauschbare Vorratsbehälter eingeschoben und benutzt werden. Der Vorratsbehälter enthält die erfindungsgemäße wässerige Aerosolzubereitung.

Der Zerstäubungsvorgang wird durch leichtes Eindrücken der Auslösetaste eingeleitet. Dabei gibt das Sperrwerk den Weg für das Abtriebsteil frei. Die gespannte Feder schiebt den Kolben in den Zylinder des Pumpengehäuses hinein. Das Fluid tritt aus der Düse des Zerstäubers in zerstäubter Form aus.

Weitere konstruktive Details sind in den PCT-Anmeldungen WO 97/12683 und WO 97/20590 offenbart, auf die hiermit inhaltlich Bezug genommen wird.

Die Bauteile des Zerstäubers (Verneblers) sind aus einem der Funktion entsprechend geeignetem Material. Das Gehäuse des Zerstäubers und - so weit es die Funktion erlaubt - auch andere Teile sind bevorzugt aus Kunststoff, z.B. im Spritzgießverfahren, hergestellt. Für medizinische Zwecke werden physiologisch unbedenkliche Materialien verwendet.

In den Figuren 6 a/b der WO 97/12687 einschließlich der dazugehörigen Beschreibung, auf die an dieser Stelle nochmals inhaltlich Bezug genommen wird, ist ein entsprechender Vernebler (Respimat®) beschrieben. Dieser eignet sich insbesondere zur Applikation der erfindungsgemäßen treibgasfreien Inhalationsaerosole.

Figur 6a zeigt der WO97/12687 zeigt einen Längsschnitt durch den Zerstäuber bei gespannter Feder, Figur 6b der WO97/12687 zeigt einen Längsschnitt durch den Zerstäuber bei entspannter Feder: Das Gehäuseoberteil (51) enthält das Pumpengehäuse (52), an dessen Ende der Halter (53) für die Zerstäuberdüse angebracht ist. In dem Halter befindet sich der Düsenkörper (54) und ein Filter (55). Der im Abtriebsflansch (56) des:Sperrspannwerkes befestigte Hohlkolben (57) ragt teilweise in den Zylinder des Pumpengehäuses hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespannter Feder anliegt. Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseobereil endet im Mundstück (65) und ist mit der aufsteckbaren Schutzkappe (66) verschlossen. Das Federgehäuse (67) mit Druckfeder (68) ist mittels der Schnappnasen (69) und Drehlager am Gehäuseoberteil drehbar gelagert. Über das Federgehäuse ist das Gehäuseunterteil (70) geschoben. Innerhalb des Federgehäuses befindet sich der austauschbare Vorratsbehälter (71) für das zu zerstäubende Fluid (72). Der Vorratsbehälter ist mit dem Stopfen (73) verschlossen, durch den der Hohlkolben in den Vorratsbehälter hineinragt und mit seinem Ende in das Fluid (Vorrat an Wirkstofflösung) eintaucht. In der Mantelfläche des Federgehäuses ist die Spindel (74) für das mechanische Zählwerk angebracht. An dem Ende der Spindel, das dem Gehäuseoberteil zugewandt ist, befindet das Antriebsritzel (75). Auf der Spindel sitzt der Reiter (76).

Wird die erfindungsgemäße Formulierung mittels der vorstehend beschriebenen Technik (Respimat®) vernebelt, sollte die ausgebrachte Masse bei wenigstens 97%, bevorzugt wenigstens 98% aller Betätigungen des Inhalators (Hube) einer definierten Menge mit einem Toleranzbereichs von maximal 25%, bevorzugt 20% dieser Menge entsprechen. Bevorzugt werden pro Hub zwischen 5 und 30 mg Formulierung als definierte Masse ausgebracht, besonders bevorzugt zwischen 5 und 20 mg.

Die erfindungsgemäße Formulierung kann jedoch auch mittels anderer als der vorstehend beschriebenen Inhalatoren, beispielsweise Jet-Stream-Inhalatoren oder anderen stationären Verneblern vernebelt werden.

Dementsprechend betrifft ein weiterer Aspekt der vorliegenden Erfindung Arzneimittel in Form von wie vorstehend beschriebenen treibgasfreien Inhaltionslösungen oder Suspensionen in Verbindung mit einer zur Verabreichung dieser Formulierungen geeigneten Vorrichtung, bevorzugt in Verbindung mit dem Respimat®. Bevorzugt zielt die vorliegende Erfindung auf treibgasfreie Inhaltionslösungen oder Suspensionen, enthaltend eines der erfindungsgemäßen Pulver, in Verbindung mit der unter der Bezeichnung Respimat® bekannten Vorrichtung. Ferner betrifft die vorliegende Erfindung vorstehend genannte Vorrichtungen zur Inhalation, bevorzugt den Respimat®, dadurch gekennzeichnet, dass sie vorstehend beschriebene erfindungsgemäße treibgasfreie Inhalationslösungen oder Suspensionen enthalten.

Erfindungsgemäß bevorzugt sind Inhalationslösungen, enthaltend eines der hier beschriebenen erfinddungsgemäßen Pulver, in einer einzigen Darreichungsform enthalten.

Die erfindungsgemäßen treibgasfreien Inhalationslösungen oder Suspensionen können neben den vorstehend, zur Applikation im Respimat® vorgesehenen Lösungen und Suspensionen auch als Konzentrate oder sterile gebrauchsfertige Inhalationslösungen bzw. -suspensionen vorliegen. Aus den Konzentraten lassen sich beispielsweise durch Zugabe von isotonischen Kochsalzlösungen gebrauchsfertige Formulierungen generieren. Sterile gebrauchsfertige Formulierungen können mittels energiebetriebener Stand- oder transportabler Vernebler, die inhalierbare Aerosole mittels Ultraschall oder Druckluft nach dem Venturiprinzip oder anderen Prinzipien erzeugen, appliziert werden.

Dementsprechend betrifft ein weiterer Aspekt der vorliegenden Erfindung Arzneimittel in Form von wie vorstehend beschriebenen treibgasfreien Inhaltionslösungen oder Suspensionen, die als Konzentrate oder sterile gebrauchsfertige Formulierungen vorliegen, in Verbindung mit einer zur Verabreichung dieser Lösungen geeigneten Vorrichtung, dadurch gekennzeichnet, daß es sich bei dieser Vorrichtung um einen energiebetriebenen Stand- oder transportablen Vernebler handelt, der inhalierbare Aerosole mittels Ultraschall oder Druckluft nach dem Venturiprinzip oder anderen Prinzipien erzeugt.

Weitere geeignete Vernebler für die inhalative Applikation von rekonstituierten Aerosolen sind der AERx™ (Aradigm), der Ultravent® (Mallinkrodt) und der Aconll® (Maquest Medical Products).

### Beispiele:

Die folgenden Beispiele dienen einer weitergehenden Erläuterung der vorliegenden Erfindung, ohne den Umfang der Erfindung allerdings auf die nachfolgenden beispielhaften Ausführungsformen zu beschränken.

### Materialien:

IgG1 ist ein humanisierter monoklonaler Antikörper mit einem Molekulargewicht von ca. 148 kDa. Der Antikörper ist von einem murinen Antikörper abgeleitet, in dem die komplementärbestimmenden Regionen des murinen Antikörpers auf ein humanes Immunglobulingerüst übertragen wurden. Es entstand ein chimärer Antikörper mit 95% humanen und 5% murinen Anteil. Der Antikörper wird aus murinen Myelomazelllinien exprimiert. Die Zellen werden mit Hilfe von Tangential Fluss Microflitration entfernt und die zellfreie Lösung durch verschiedene Chromatographiemethoden aufgereinigt. Weitere Schritte umfassen eine Nucleasebehandlung, Behandlung bei niedrigem pH-Wert und eine Nanofiltration. Die Bulklösung enthält Histidin und Glycin als Puffer und wurde für die Herstellung der Lösung zur Sprühtrocknung mittels Diafiltration auf ca. 100mg/ml aufkonzentriert. Der Bulk für die Herstellung der Sprühlösung besaß 0,6% Aggregate. Im Handel ist der Antikörper als steriles, lyophilisiertes Produkt zur intramuskulären Applikation. Das Fertigarzneimittel ist bei 2-8°C mindestens 2 Jahre haltbar.

### Sprühtrocknung mit Büchi B-290:

Die Sprühtrocknung der hergestellten Lösungen bestehend aus IgG1/Mannitol wurden mit Hilfe eines Büchi Mini Spray Dryer B-290 der Fa. Büchi Labortechnik (AG, CH) durchgeführt.

Der Sprühtrockner ist aus einem Heizsystem, einem Filter, einem Aspirator, einem Trocknungsturm, einem Zyklon, Temperatursensoren zur Messung der Einlass- und der Auslasstemperatur und einem Auffanggefäß aufgebaut. Mit Hilfe einer peristaltischen Pumpe wird die zu versprühende Lösung in die Zwei-Stoffdüse gepumpt. Dort findet die Zerstäubung der Lösung in kleine Tropfen statt, die im Sprühturm getrocknet werden. Die Trocknung im Sprühturm erfolgt durch erwärmte Luft, die im Gleichstromverfahren mit Hilfe des Aspirators durch den Sprühturm gesaugt wird. Das Produkt wird im Auffanggefäß nach Passieren des Zyklons aufgefangen

Der Feststoffanteil der Sprühlösungen betrug 10% (w/V) in 100 ml versprühter Lösung. Die Einlasstemperatur betrug 90°C, die Liquid-Feed-Rate ca. 3 ml/min, die Aspirator-Flow-Rate 35 m³/h und die Zerstäubungsflussrate 0,67 m³/h. Daraus ergibt sich eine Auslasstemperatur von 50-55°C.

### Größenausschlußchromatographie (SEC-HPLC):

Die Größenausschlußchromatographie wurde verwendet, um lösliche Aggregate in den Sprühlösungen und in den rekonstituierten Pulvern zu bestimmen. Die SEC-HPLC wurde mit einer HP1090 der Fa. Agilent (Waldbronn, DE) durchgeführt. Als Säule wurde eine TSK3000SWXL Säule (300 x 7.8mm) der Fa. Tosoh Biosep (Stuttgart, DE) verwendet. Der Puffer aus 0.1 M di-Natriumhydrogenphosphat-Dihydrat, 0.1M Natriumsulfat entwässert und wurde mit ortho-Phosphorsäure 85% auf pH 6.8 eingestellt. Die aufgegebene Probenmenge betrug 25µl bei einer Proteinkonzentration zwischen 2-10 mg/ml. Die Detektion des Proteins erfolgte mit Hilfe eines Dioden-Array Detektors bei 280nm. Für die Auswertung der Chromatogramme wurde die Software Chemstation von Agilent verwendet.

### Vakuumtrocknung:

Die Vakuumtrocknung wurde in einem Vakuumtrockenschrank durchgeführt. Die Temperatur wurde auf 32°C, das Vakuum auf ungefähr 0,1 mbar eingestellt. Die Proben wurden in 2R-Vials zum Trocknen in den Schrank gestellt, dabei wurden die Stopfen ganz entfernt und zur Trocknung ebenfalls in den Trockenschrank gelegt. Die Trocknungsdauer betrug 24 Std.

### Test zur Bestimmung der Lagerstabilität:

Die Proben wurden nach der Sprühtrocknung in Klimaschränke eingelagert. Die Bedingungen waren 2-8°C, 25°C/60% rF, 40°C/75%rF. Die Feuchten wurden mit entsprechenden gesättigten Lösungen eingestellt, für 75%rF wurde Natriumchlorid verwendet und für 60%rF Ammoniumnitrat. Die Proben wurden teilweise offen, aber auch verbördelt und in Aluminiumbeutel eingeschweißt, eingelagert. Die Probenziehung erfolgte nach 3 Tagen, 1, 4, 15 und 52 Wochen. Nach 3 Tagen wurden nur offenen Proben, nach 1, und 4 Wochen wurden offene und geschlossene Proben, und nach 15 und 52 Wochen nur geschlossenen Proben entnommen. Die anschließende Analytik zur Untersuchung auf Aggregate wurde mit SEC-HPLC durchgeführt.

### Wassergehaltsbestimmung nach Karl-Fischer:

Der Restwassergehalt in den getrockneten Produkten wurde mittels coulometrischer Titration (Metrohm 737 KF Coulometer mit 703 Titrationsstand, Filderstadt, DE) bestimmt. Zur Bestimmung wurde Pulver Methanol (Hydranal - Methanol dry, VWR / Merck Eurolab, Darmstadt, DE) aufgelöst bzw. dispergiert. Die Messlösung (Hydranal -Coulomat- Lösung, VWR / Merck Eurolab, Darmstadt, DE) des Metrohm Coulometers wurde zu Beginn der Messungen konditioniert, d.h. die Messlösung wurde auf einen Nullgehalt an Wasser austitriert. Die Probe wurde in die Titrationszelle injiziert.

### Röntgendiffraktometrie (Weitwinkelröntgendiffraktometrie (WAXS)):

Um die Kristallinität der getrockneten Proben zu bestimmen, wurden die Proben mit einem Seifert, X- Ray Diffractometer XRD 3000 TT (Fa. Seifert, Ahrensburg, DE) in einem auf 22 °C temperierten Raum untersucht. Die Röntgenröhre Cu- Anode, Cu-Kα-Strahlung mit λ = 0,15418 mm (Primärfilter Ni), wurde mit einer Anodenspannung von 40 kV und einer Stromstärke von 30 mA betrieben. Nach Positionieren des Probentellers im Gerät wurde die Probe im Bereich von 5 bis 40° mit einer Scanrate von 2 θ = 0,05° mit 2 sec Messdauer bei jedem Winkel vermessen.

Die Pulverdiffraktogramme wurden mit der ScanX- Rayflex Applikation, Version 3.07 device XRD 3000 (Scan), bzw. der Rayflex Version 2.1, 1996 (Analyse) am Detektor SC 1000 V aufgenommen.

### Bestimmung der mittleren Teilchengröße der Partikel:

Die mittlere Teilchengrößen der Partikel wurde mit Hilfe des Sympatech Helos der Fa Sympatech GmbH (Clausthal-Zellerfeld, DE) bestimmt. Das Messprinzip beruht auf Laserbeugung, verwendet wird ein Helium-Neon-Laser. 1-3 mg Pulver werden mit einem Luftdruck von 2bar dispergiert, und vor der Fourierlinse (50mm) durch einen parallelen Laserstrahl geführt. Die Partikelgrößenverteilung wird mit einem Fraunhofer-Modell ausgewertet.

### Bestimmung des aerodynamischen Durchmessers der Partikel:

Der aerodynamische Teilchendurchmesser wurde mit Hilfe des APS 3321 (Aerodynamic Particle Sizer) der Fa. TSI GmbH, Particle Instruments (Aachen, DE) bestimmt. Das zugrunde liegende Messprinzip ist eine Time-of-Flight-Messung, wobei die Partikel durch eine Düse beschleunigt werden. Hierzu werden die Formulierungen in Gelatinekapseln abgefüllt und mit Hilfe des Handihalers®, durch eine Volumenstrom von 39ml/min, der durch eine Vakuumpumpe erzeugt wird, in das Gerät eingebracht und auf der Prallplatte und dem Filter abgeschieden. (Ausbringzeit 6,15s, Einstellungen des Gerätes Delta P Aerosol auf ca. 0,47 inch W.C., während der Ausbringung zwischen 0,2-0,8 inches W.C)

### Bestimmung der ausgebrachten Menge:

Die ausgebrachte Menge wird mit Hilfe des APS 3321 Aerodynamic Particle Sizer) der Fa. TSI GmbH, Particle Instruments (Aachen, DE) bestimmt. Das Pulver wird mit einem Volumenstrom von 39 ml/min aus dem Handihaler ausgebracht, und schlägt sich auf der Prallplatte und einem Filter nieder. Die Kapseln werden nach der Ausbringung gewogen und dadurch die ausgebrachte Menge bestimmt. Die ausgebrachte Menge ergibt sich als Differenz der Einwaage des Pulvers abzüglich des verbleibenden Rests an Pulver in der Kapsel nach Ausbringung.

### Beispiel 1

### 30/70 hulgG1/Mannitol zur pulmonalen Anwendung

Der vorhandene Bulk des Antikörpers wurde mit Mannitol im entsprechenden Verhältnis versetzt und gesprühtrocknet. Eine 30%-ige hulgG1-Lösung mit 70% Mannitol wurden erhalten, indem 27,5ml Bulklösung (c=109 mg/ml) mit 7,0 g Mannitol ad 100 ml Wasser aufgefüllt wurden. Der Feuchtigkeitsgehalt der Formulierung direkt nach der Sprühtrocknung war 4,8% und wurde durch die anschließende Vakuumnachtrocknung auf 0,9% gesenkt. 50 mg Pulver wurden in 5 ml Wasser für SEC-HPLC aufgelöst. Die Menge an Aggregaten mit HPSEC bestimmt, betrug nach der Sprühtrocknung 5,1%, nach der Nachtrocknung 8,8%. Bei Lagerung bei 2-8°C war die Menge an Aggregaten nach 4 Wochen 9,3% und nach 15 Wochen 9,5%. Die Menge der Aggregate stieg nach einer Lagerdauer von 4 Wochen bei 25°C/60%rF auf 10,3% an, nach 15 Wochen auf 10,5%. Der Aggregatgehalt betrug nach der Lagerung 4 Wochen bei 40°C/75%rF 15,7%, nach 15 Wochen 15,3%.

### Beispiel 2

### 40/60 hulgG1/Mannitol zur pulmonalen Anwendung

Der vorhandene Bulk des Antikörpers wurde mit Mannitol im entsprechenden Verhältnis versetzt und nach der hier beschriebenen Methode gesprühtrocknet. Eine 40%-ige hulgG1-Lösung mit 60% Mannitol wurden erhalten, indem 36,7 ml Bulklösung (c=109 mg/ml) mit 6,0 g Mannitol ad 100 ml Wasser aufgefüllt wurden. Der Feuchtigkeitsgehalt der Formulierung direkt nach der Sprühtrocknung war 5,0% und wurde durch die anschließende Vakuumnachtrocknung auf 0,9% gesenkt. 50 mg Pulver wurden in 5 ml Wasser für SEC-HPLC aufgelöst. Die Menge an Aggregaten mit HPSEC bestimmt, betrug nach der Sprühtrocknung 1,1%, nach der Nachtrocknung 1,5%. Bei Lagerung bei 2-8°C war die Menge an Aggregaten nach 4 Wochen 9,5% und nach 15 Wochen 9,1%. Die Menge der Aggregate stieg nach einer Lagerdauer von 4 Wochen bei 25°C/60%rF auf 8,1% an, nach 15 Wochen auf 7,4%. Der Aggregatgehalt betrug nach der Lagerung 4 Wochen bei 40°C/75%rF 11,6%, nach 15 Wochen 13,3%. Der MMAD lag bei 7,4 µm und die ausgebrachte Menge betrug 74,1%.

### Beispiel 3

### 60/40 hulgG1/Mannitol zur pulmonalen Anwendung

Der vorhandene Bulk des Antikörpers wurde mit Mannitol im entsprechenden Verhältnis versetzt und nach der hier beschriebenen Methode gesprühtrocknet. Eine 60%-ige hulgG1-Lösung mit 40% Mannitol wurden erhalten, indem 55,1 ml Bulklösung (c=109 mg/ml) mit 4,0g Mannitol ad 100 ml Wasser aufgefüllt wurden. Der Feuchtigkeitsgehalt der Formulierung direkt nach der Sprühtrocknung war 5,4% und wurde durch die anschließende Vakuumnachtrocknung auf 0,7% gesenkt. 50 mg Pulver wurden in 5 ml Wasser für SEC-HPLC aufgelöst. Die Menge an Aggregaten betrug nach der Sprühtrocknung 1,1%, nach der Nachtrocknung 1,5%. Bei Lagerung bei 2-8°C war die Menge an Aggregaten nach 4 Wochen 1,8% und nach 15 Wochen 1,4%. Die Menge der Aggregate stieg nach einer Lagerdauer von 4 Wochen bei 25°C/60%rF auf 2,5% an, nach 15 Wochen auf 2,7%. Der Aggregatgehalt betrug nach der Lagerung 4 Wochen bei 40°C/75%rF 3,3%, nach 15 Wochen 3,2%. Der MMAD lag bei 7,5 µm. und die ausgebrachte Menge betrug 85,7%.

### Beispiel 4

### 70/30 hulgG1/Mannitol zur pulmonalen Anwendung

Der vorhandene Bulk des Antikörpers wurde mit Mannitol im entsprechenden Verhältnis versetzt und nach der beschriebenen Methode gesprühtrocknet. Eine 70%-ige hulgG1-Lösung mit 30% Mannitol wurden erhalten, indem 64,2 ml Bulklösung (c=109 mg/ml) mit 3,0 g Mannitol ad 100 ml Wasser aufgefüllt wurden. Der Feuchtigkeitsgehalt der Formulierung direkt nach der Sprühtrocknung war 7,0% und wurde durch die anschließende Vakuumnachtrocknung auf 0,7% gesenkt. 50 mg Pulver wurden in 5 ml Wasser für SEC-HPLC aufgelöst. Die Menge an Aggregaten betrug nach der Sprühtrocknung 0,6%, nach der Nachtrocknung 0,9%. Bei Lagerung bei 2-8°C war die Menge an Aggregaten nach 4 Wochen 0,7% und nach 15 Wochen 0,7%. Die Menge der Aggregate stieg nach einer Lagerdauer von 4 Wochen bei 25°C/60%rF auf 1,7% an, nach 15 Wochen auf 1,9%. Der Aggregatgehalt betrug nach der Lagerung 4 Wochen bei 40°C/75%rF 2,2%, nach 15 Wochen 3,3%. Der MMAD lag bei 5,7 µm und die ausgebrachte Menge betrug 80,2%

### Beispiel 5

### 80/20 hulgG1/Mannitol zur pulmonalen Anwendung

Der vorhandene Bulk des Antikörpers wurde mit Mannitol im entsprechenden Verhältnis versetzt und nach der hier beschriebenen Methode gesprühtrocknet. Eine 80%-ige hulgG1-Lösung mit 20% Mannitol wurden erhalten, indem 73,4 ml Bulklösung (c=109 mg/ml) mit 2,0 g Mannitol ad 100 ml Wasser aufgefüllt wurden Der Feuchtigkeitsgehalt der Formulierung direkt nach der Sprühtrocknung war 6,7% und wurde durch die anschließende Nachtrocknung auf 0,6% gesenkt. Die Menge an Aggregaten betrug nach der Sprühtrocknung 0,6%, nach der Nachtrocknung 0,7%. 50 mg Pulver wurden in 5 ml Wasser für SEC-HPLC aufgelöst. Bei Lagerung bei 2-8°C war die Menge an Aggregaten nach 4 Wochen 0,7% und nach 15 Wochen 0,7%. Die Menge der Aggregate stieg nach einer Lagerdauer von 4 Wochen bei 25°C/60%rF auf 1,7% an, nach 15 Wochen auf 2,0%. Der Aggregatgehalt betrug nach der Lagerung 4 Wochen bei 40°C/75%rF 2,3%, nach 15 Wochen 3,5%. Der MMAD lag bei 5,5 µm und die ausgebrachte Menge betrug 72,4%

### Beispiel 6

### 100/0 hulgG1/Mannitol zur pulmonalen Anwendung

Der vorhandene Bulk des Antikörpers wurde ohne Zugabe eines Hilfsstoffs nach der hier beschriebenen Methode gesprühtrocknet. Der Feuchtigkeitsgehalt der Formulierung direkt nach der Sprühtrocknung war 9,1% und wurde durch die anschließende Vakuumnachtrocknung auf 1,0% gesenkt. 50 mg Pulver wurden in 5 ml Wasser für SEC-HPLC aufgelöst. Die Menge an Bei Lagerung bei 2-8°C war die Menge an Aggregaten nach 4 Wochen 3,7% und nach 15 Wochen 4,2%. Aggregaten betrug nach der Sprühtrocknung 2,7%, nach der Nachtrocknung 2,7%. Die Menge der Aggregate stieg nach einer Lagerdauer von 4 Wochen bei 25°C/60%rF auf 6,1% an, nach 15 Wochen auf 8,7%. Der Aggregatgehalt betrug nach der Lagerung 4 Wochen bei 40°C/75%rF %, nach 15 Wochen 12,1%.

### Beispiel 7

### Herstellung von weiteren erfindungsgemäßen Pulvern

### 60/40 hulgG1/Mannitol zur pulmonalen Anwendung

Der vorhandene Bulk des Antikörpers wird mit Mannitol im entsprechenden Verhältnis versetzt und nach der hier beschriebenen Methode gesprühtrocknet und nachgetrocknet. Eine 60%-ige hulgG1-Lösung mit 40% Mannitol wird erhalten, indem 13,76 ml Bulklösung (c=109 mg/ml) mit 1,0 g Mannitol ad 100 ml Wasser aufgefüllt werden. Der Feststoffanteil der Lösung beträgt 2,5% (w/V).

### 70/30 hulgG1/Mannitol zur pulmonalen Anwendung

Der vorhandene Bulk des Antikörpers wird mit Mannitol im entsprechenden Verhältnis versetzt und nach der beschriebenen Methode gesprühtrocknet und nachgetrocknet. Eine 70%-ige hulgG1-Lösung mit 30% Mannitol wird erhalten, indem 16,05 ml Bulklösung (c=109 mg/ml) mit 0,75 g Mannitol ad 100 ml Wasser aufgefüllt werden. Der Feststoffanteil der Lösung beträgt 2,5% (wN).

### 70/25/5 hulgG1/Mannitol/Isoleucin zur pulmonalen Anwendung

Der vorhandene Bulk des Antikörpers wird mit Mannitol und Isoleucin im entsprechenden Verhältnis versetzt und nach der beschriebenen Methode gesprühtrocknet und nachgetrocknet. Eine 70%-ige hulgG1-Lösung mit 25% Mannitol und 5% Isoleucin wird erhalten, indem 16,05 ml Bulklösung (c=109 mg/ml) mit 0,625g Mannitol und 0,125g Isoleucin ad 100 ml Wasser aufgefüllt werden. Der Feststoffanteil der Lösung beträgt 2,5% (w/V).

### 60/30/10 hulgG1/Mannitol/Isoleucin zur pulmonalen Anwendung

Der vorhandene Bulk des Antikörpers wird mit Mannitol und Isoleucin im entsprechenden Verhältnis versetzt und nach der beschriebenen Methode gesprühtrocknet und nachgetrocknet. Eine 60%-ige hulgG1-Lösung mit 30% Mannitol und 10% Isoleucin wird erhalten, indem 13,76 ml Bulklösung (c=109mg/ml) mit 0,75 g Mannitol und 0,25 g Isoleucin ad 100ml Wasser aufgefüllt werden. Der Feststoffanteil der Lösung beträgt 2,5% (wN).

### 70/25/5 hulgG1/Mannitol/Tri-Isoleucin zur pulmonalen Anwendung

Der vorhandene Bulk des Antikörpers wird mit Mannitol und Tri-Isoleucin im entsprechenden Verhältnis versetzt und nach der beschriebenen Methode gesprühtrocknet und nachgetrocknet. Eine 70%-ige hulgG1-Lösung mit 25% Mannitol und 5% Tri-Isoleucin wird erhalten, indem 16,05 ml Bulklösung (c=109 mg/ml) mit 0,625 g Mannitol und 0,125g Tri-Isoleucin ad 100ml Wasser aufgefüllt wurden. Der Feststoffanteil der Lösung beträgt 2,5% (w/V).

### 60/30/10 hulgG1/Mannitol/Tri-Isoleucin zur pulmonalen Anwendung

Der vorhandene Bulk des Antikörpers wird mit Mannitol und Tri-Isoleucin im entsprechenden Verhältnis versetzt und nach der beschriebenen Methode gesprühtrocknet und nachgetrocknet. Eine 60%-ige hulgG1-Lösung mit 30% Mannitol und 10% Tri-Isoleucin wird erhalten, indem 13,76 ml Bulklösung (c=109 mg/ml) mit 0,75 g Mannitol und 0,25g Tri-Isoleucin ad 100ml Wasser aufgefüllt werden. Der Feststoffanteil der Lösung betrug 2,5% (w/V).

## Patentansprüche

1. Sprühgetrocknetes amorphes Pulver enthaltend einen pharmazeutischen Wirkstoff und einen Matrixbildner ***dadurch gekennzeichnet,* dass** das Pulver eine Restfeuchte von weniger als 1,2% (w/w) aufweist und wobei das Pulver als einen oder mehrere zusätzliche Hilfsstoffe Tripeptide der allgemeinen Formel Ile-X-X, X-IIe-X, X-X-Ile enthält, wobei X eine der folgenden Aminosäuren sein kann: Alanin, Glycin, Arginin, Histidin, Glutaminsäure, Glutamin, Asparagin, Asparaginsäure, Cystein, Leucin, Lysin, Isoleucin (Ile), Valin, Tryptophan, Methionin, Phenylalanin, Prolin, Serin, Threonin, Tyrosin, L-Aspartyl-L-phenylalanin-methylester (= Aspartam), Trimethylammonio-acetat und wobei Ile für Isoleucin steht.

2. Sprühgetrocknetes Pulver nach Anspruch 1 ***dadurch gekennzeichnet,* dass** es sich bei dem Tripeptid um ein Tri-Peptid der Formel (Ile)₂-X handelt, beispielsweise Ile―Ile-X, Ile-X-Ile, oder X-Ile-Ile, wobei X eine der folgenden Aminosäuren sein kann: Alanin, Glycin, Arginin, Histidin, Glutaminsäure, Glutamin, Asparagin, Asparaginsäure, Cystein, Leucin, Lysin, Isoleucin (Ile), Valin, Tryptophan, Methionin, Phenylalanin, Prolin, Serin, Threonin, Tyrosin, L-Aspartyl-L-phenylalanin-methylester (= Aspartam), Trimethylammonio-acetat und wobei Ile für Isoleucin steht.

3. Sprühgetrocknetes Pulver nach Anspruch 1 oder 2 ***dadurch gekennzeichnet,* dass** es sich bei dem Tripeptid um Ile-Ile-Gly, Ile-Ile-Ala, Ile-Ile-Val, Ile-Ile-Ile, Ile-Ile-Met, Ile-Ile-Pro, Ile-Ile-Phe, Ile-Ile-Trp, Ile-Ile-Ser, Ile-Ile-Thr, Ile-Ile-Cys, Ile-Ile-Tyr, Ile-Ile-Asp, Ile-Ile-Glu, Ile-Ile-Lys, Ile-Ile-Arg, Ile-Ile-His, Ile-Gly-Ile, Ile-Ala-Ile, Ile-Val-Ile, Ile-Met-Ile, Ile-Pro-Ile, Ile-Phe-Ile, Ile-Trp-Ile, Ile-Ser-Ile, Ile-Thr-Ile, Ile-Cys-Ile, Ile-Tyr-Ile, Ile-Asp-Ile, Ile-Glu-Ile, Ile-Lys-Ile, Ile-Arg-Ile, Ile-His-Ile handelt.

4. Sprühgetrocknetes Pulver nach einem der Ansprüche 1 bis 3 ***dadurch gekennzeichnet,* dass** es sich bei dem Tripeptid um Tri-Isoleucin (Ile-Ile-Ile) handelt.

5. Sprühgetrocknetes Pulver nach einem der Ansprüche 1 bis 4, ***dadurch gekennzeichnet,* dass** das Pulver aus feinteiligen inhalierbaren Partikeln mit einem mittleren aerodynamischen Teilchendurchmesser (=MMAD) und/oder einem mittleren Teilchendurchmesser (=MMD) von kleiner 10 µm bestehen.

6. Sprühgetrocknetes Pulver nach einem der Ansprüche 1 bis 5, ***dadurch gekennzeichnet,* dass** es sich bei dem Matrixbildner um Zucker, Polyole, Polymere oder eine Kombination aus diesen handelt.

7. Sprühgetrocknetes Pulver nach Anspruch 6 ***dadurch gekennzeichnet,* dass** es sich bei dem Zucker um ein Mono-, Di-, Oligo- oder Polysaccharid oder um eine Kombination hieraus handelt.

8. Sprühgetrocknetes Pulver nach Anspruch 6 ***dadurch gekennzeichnet,* dass** es sich bei dem Polyol um Mannitol handelt.

9. Sprühgetrocknetes Pulver nach Anspruch 8 ***dadurch gekennzeichnet,* dass** der Anteil an Mannitol zwischen 20 und 60% (w/w) der Trockenmasse des Pulvers beträgt.

10. Sprühgetrocknetes Pulver nach Anspruch 8 ***dadurch gekennzeichnet,* dass** der Anteil an Mannitol zwischen 25 und 50% (w/w) der Trockenmasse des Pulvers beträgt.

11. Sprühgetrocknetes Pulver nach Anspruch 8 ***dadurch gekennzeichnet,* dass** der Anteil an Mannitol zwischen 30 und 40% (w/w) der Trockenmasse des Pulvers beträgt.

12. Sprühgetrocknetes Pulver nach einem der Ansprüche 1 bis 11 ***dadurch gekennzeichnet,* dass** es sich bei dem pharmazeutischen Wirkstoff um ein biologisches Makromolekül handelt.

13. Sprühgetrocknetes Pulver nach Anspruch 12 ***dadurch gekennzeichnet,* dass** es sich bei dem biologischen Makromolekül um ein Polypeptid oder Protein handelt.

14. Sprühgetrocknetes Pulver nach Anspruch 13 ***dadurch gekennzeichnet,* dass** es sich bei dem Polypeptid oder Protein um einen Wachstumsfaktor, ein Enzym oder einen Antikörper handelt.

15. Sprühgetrocknetes Pulver nach Anspruch 14 ***dadurch gekennzeichnet,* dass** der Anteil der im Pulver in aggregierter Form vorliegenden Wachstumsfaktoren, Enzyme oder Antikörper unterhalb von 3,5% (w/w) liegt.

16. Pharmazeutische Zusammensetzung enthaltend ein sprühgetrocknetes Pulver nach einem der Ansprüche 5 bis 15.

17. Pharmazeutischen Zusammensetzung nach Anspruch 16 zur inhalativen Applikation ***dadurch gekennzeichnet, dass*** es sich bei der pharmazeutischen Zusammensetzung um treibgashaltige Dosieraerosole oder um treibgasfreie Inhalationslösungen handelt.

18. Verfahren zur Herstellung eines sprühgetrockneten Pulvers nach einem der Ansprüche 1 bis 15 ***dadurch gekennzeichnet,* dass** eine Sprühlösung enthaltend zumindest einen pharmazeutischen Wirkstoff und einen Matrixbildner
a) unterhalb einer Temperatur von 200/120°C versprüht wird; u n d
b) das resultierende Pulver anschließend unter Vakuum bei einer Temperatur oberhalb von 15°C und unterhalb von 60°C nachgetrocknet wird.

19. Verfahren nach Anspruch 18 ***dadurch gekennzeichnet,* dass** die Nachtrocknung bei einem Druck zwischen 0,01 und 10 mbar durchgeführt wird.

20. Verfahren nach Anspruch 18 oder 19 ***dadurch gekennzeichnet,* dass** das sprühgetrocknete Pulver für zumindest 12 Std. nachgetrocknet wird.

21. Verfahren zur Herstellung eines sprühgetrockneten Pulvers nach einem der Ansprüche 1 bis 15 ***dadurch gekennzeichnet,* dass** eine Sprühlösung enthaltend zumindest einen pharmazeutischen Wirkstoff und einen Matrixbildner
a) unterhalb einer Temperatur von 105/60°C versprüht wird; u n d
b) das resultierende Pulver anschließend unter Vakuum bei einem Druck von ca. 0,05 bis 0,2 mbar, bei einer Temperatur oberhalb von 30°C und unterhalb von 60°C für ungefähr 18 bis 30 Std. nachgetrocknet wird.

## Claims

1. Spray-dried amorphous powder comprising a pharmaceutical active substance and a matrix-forming agent, **characterised in that** the powder has a residual moisture content of less than 1.2% (w/w), and the powder comprises, as one or more additional adjuvants, tripeptides of general formula Ile-X-X, X-Ile-X, X-X-Ile, wherein X may be one of the following amino acids: alanine, glycine, arginine, histidine, glutamic acid, glutamine, asparagine, aspartic acid, cysteine, leucine, lysine, isoleucine (Ile), valine, tryptophan, methionine, phenylalanine, proline, serine, threonine, tyrosine, L-aspartyl-L-phenylalanine-methyl ester (= aspartame), trimethylammonium acetate and wherein Ile denotes isoleucine.

2. Spray-dried powder according to claim 1, **characterised in that** the tripeptide is a tripeptide of the formula (Ile)₂-X, for example Ile-Ile-X, Ile-X-Ile or X-Ile-Ile, wherein X may be one of the following amino acids: alanine, glycine, arginine, histidine, glutamic acid, glutamine, asparagine, aspartic acid, cysteine, leucine, lysine, isoleucine (Ile), valine, tryptophan, methionine, phenylalanine, proline, serine, threonine, tyrosine, L-aspartyl-L-phenylalanine-methyl ester (= aspartame), trimethylammonium acetate and wherein Ile denotes isoleucine.

3. Spray-dried powder according to claim 1 or 2, **characterised in that** the tripeptide is Ile-Ile-Gly, Ile-Ile-Ala, Ile-Ile-Val, Ile-Ile-Ile, Ile-Ile-Met, Ile-Ile-Pro, Ile-Ile-Phe, Ile-Ile-Trp, Ile-Ile-Ser, Ile-Ile-Thr, Ile-Ile-Cys, Ile-Ile-Tyr, Ile-Ile-Asp, Ile-Ile-Glu, Ile-Ile-Lys, Ile-Ile-Arg, Ile-Ile-His, Ile-Gly-Ile, Ile-Ala-Ile, Ile-Val-Ile, Ile-Met-Ile, Ile-Pro-Ile, Ile-Phe-Ile, Ile-Trp-Ile, Ile-Ser-Ile, Ile-Thr-Ile, Ile-Cys-Ile, Ile-Tyr-Ile, Ile-Asp-Ile, Ile-Glu-Ile, Ile-Lys-Ile, Ile-Arg-Ile, Ile-His-Ile.

4. Spray-dried powder according to one of claims 1 to 3, **characterised in that** the tripeptide is tri-isoleucine (Ile-Ile-Ile).

5. Spray-dried powder according to one of claims 1 to 4, **characterised in that** the powder consists of finely divided inhalable particles with a mass median aerodynamic diameter (=MMAD) and/or a mass median diameter (=MMD) of less than 10 µm.

6. Spray-dried powder according to one of claims 1 to 5, **characterised in that** the matrix-forming agent is a sugar, polyol, polymer or a combination thereof.

7. Spray-dried powder according to claim 6, **characterised in that** the sugar is a mono-, di-, oligo- or polysaccharide or a combination thereof.

8. Spray-dried powder according to claim 6, **characterised in that** the polyol is mannitol.

9. Spray-dried powder according to claim 8, **characterised in that** the proportion of mannitol is between 20 and 60% (w/w) of the dry mass of the powder.

10. Spray-dried powder according to claim 8, **characterised in that** the proportion of mannitol is between 25 and 50% (w/w) of the dry mass of the powder.

11. Spray-dried powder according to claim 8, **characterised in that** the proportion of mannitol is between 30 and 40% (w/w) of the dry mass of the powder.

12. Spray-dried powder according to one of claims 1 to 11, **characterised in that** the pharmaceutical active substance is a biological macromolecule.

13. Spray-dried powder according to claim 12, **characterised in that** the biological macromolecule is a polypeptide or protein.

14. Spray-dried powder according to claim 13, **characterised in that** the polypeptide or protein is a growth factor, an enzyme or an antibody.

15. Spray-dried powder according to claim 14, **characterised in that** the proportion of growth factors, enzymes or antibodies present in the powder in aggregated form is less than 3.5% (w/w).

16. Pharmaceutical composition comprising a spray-dried powder according to one of claims 5 to 15.

17. Pharmaceutical composition according to claim 16 for administration by inhalation, **characterised in that** the pharmaceutical composition is a propellant-comprising metered-dose aerosol or a propellant-free inhalable solution.

18. Process for preparing a spray-dried powder according to one of claims 1 to 15, **characterised in that** a spray solution comprising at least one pharmaceutical active substance and a matrix-forming agent
a) is sprayed below a temperature of 200/120°C; and
b) the resulting powder is then after-dried *in vacuo* at a temperature above 15°C and below 60°C.

19. Process according to claim 18, **characterised in that** the after-drying is carried out at a pressure of between 0.01 and 10 mbar.

20. Process according to claim 18 or 19, **characterised in that** the spray-dried powder is after-dried for at least 12 hrs.

21. Process for preparing a spray-dried powder according to one of claims 1 to 15, **characterised in that** a spray solution comprising at least one pharmaceutical active substance and a matrix-forming agent
a) is sprayed below a temperature of 105/60°C; and
b) the resulting powder is then after-dried *in vacuo* at a pressure of approx. 0.05 to 0.2 mbar, at a temperature above 30°C and below 60°C, for about 18 to 30 hrs.

## Revendications

1. Poudre amorphe séchée par dispersion contenant un principe actif pharmaceutique et un formateur de matrice **caractérisée en ce que** la poudre présente une humidité résiduelle inférieure à 1,2 % (m/m) et où la poudre contient comme un ou plusieurs adjuvants supplémentaires des tripeptides de formule générale Ile-X-X, X-Ile-X, X-X-Ile, où X peut être l'un des aminoacides suivants : alanine, glycine, arginine, histidine, acide glutamique, glutamine, asparagine, acide aspartique, cystéine, leucine, lysine, isoleucine (Ile), valine, tryptophane, méthionine, phénylalanine, proline, sérine, thréonine, tyrosine, L-aspartyl-L-phénylalanine-méthylester (= aspartame), triméthylammonioacétate et où Ile représente l'isoleucine.

2. Poudre séchée par dispersion selon la revendication 1 **caractérisée en ce que** le tripeptide est un tri-peptide de formule (Ile)₂-X, par exemple Ile-Ile-X, Ile-X-Ile ou X-Ile-Ile, où X peut être l'un des aminoacides suivants : alanine, glycine, arginine, histidine, acide glutamique, glutamine, asparagine, acide aspartique, cystéine, leucine, lysine, isoleucine (Ile), valine, tryptophane, méthionine, phénylalanine, proline, sérine, thréonine, tyrosine, L-aspartyl-L-phénylalanine-méthylester (= aspartame), triméthylammonioacétate et où Ile représente l'isoleucine.

3. Poudre séchée par dispersion selon la revendication 1 ou 2 **caractérisée en ce que** le tripeptide est Ile-Ile-Gly, Ile-Ile-Ala, Ile-Ile-Val, Ile-Ile-Ile, Ile-Ile-Met, Ile-Ile-Pro, Ile-Ile-Phe, Ile-Ile-Trp, Ile-Ile-Ser, Ile-Ile-Thr, Ile-Ile-Cys, Ile-Ile-Tyr, Ile-Ile-Asp, Ile-Ile-Glu, Ile-Ile-Lys, Ile-Ile-Arg, Ile-Ile-His, Ile-Gly-Ile, Ile-Ala-Ile, Ile-Val-Ile, Ile-Met-Ile, Ile-Pro-Ile, Ile-Phe-Ile, Ile-Trp-Ile, Ile-Ser-Ile, Ile-Thr-Ile, Ile-Cys-Ile, Ile-Tyr-Ile, Ile-Asp-Ile, Ile-Glu-Ile, Ile-Lys-Ile, Ile-Arg-Ile, Ile-His-Ile.

4. Poudre séchée par dispersion selon l'une des revendications 1 à 3 **caractérisée en ce que** le tripeptide est la tri-isoleucine (Ile-Ile-Ile).

5. Poudre séchée par dispersion selon l'une des revendications 1 à 4 **caractérisée en ce que** la poudre consiste en particules inhalables finement divisées ayant un diamètre particulaire aérodynamique moyen (=MMAD) et/ou un diamètre particulaire moyen (=MMD) inférieur à 10 µm.

6. Poudre séchée par dispersion selon l'une des revendications 1 à 5 **caractérisée en ce que** le formateur de matrice est un sucre, des polyols, des polymères ou une combinaison de ceux-ci.

7. Poudre séchée par dispersion selon la revendication 6 **caractérisée en ce que** le sucre est un mono-, di-, oligo- ou polysaccharide ou une combinaison de ceux-ci.

8. Poudre séchée par dispersion selon la revendication 6 **caractérisée en ce que** le polyol est le mannitol.

9. Poudre séchée par dispersion selon la revendication 8 **caractérisée en ce que** la proportion de mannitol est entre 20 et 60 % (m/m) de la masse sèche de la poudre.

10. Poudre séchée par dispersion selon la revendication 8 **caractérisée en ce que** la proportion de mannitol est entre 25 et 50 % (m/m) de la masse sèche de la poudre.

11. Poudre séchée par dispersion selon la revendication 8 **caractérisée en ce que** la proportion de mannitol est entre 30 et 40 % (m/m) de la masse sèche de la poudre.

12. Poudre séchée par dispersion selon l'une des revendications 1 à 11 **caractérisée en ce que** le principe actif pharmaceutique est une macromolécule biologique.

13. Poudre séchée par dispersion selon la revendication 12 **caractérisée en ce que** la macromolécule biologique est un polypeptide ou une protéine.

14. Poudre séchée par dispersion selon la revendication 13 **caractérisée en ce que** le polypeptide ou la protéine est un facteur de croissance, une enzyme ou un anticorps.

15. Poudre séchée par dispersion selon la revendication 14 **caractérisée en ce que** la proportion des facteurs de croissance, enzymes ou anticorps présents sous forme agrégée dans la poudre est inférieure à 3,5 % (m/m).

16. Composition pharmaceutique contenant une poudre séchée par dispersion selon l'une des revendications 5 à 15.

17. Composition pharmaceutique selon la revendication 16 pour l'application par inhalation **caractérisée en ce que** la composition pharmaceutique représente des aérosols de dosage contenant des gaz propulseurs ou des solutions pour l'inhalation sans gaz propulseur.

18. Procédé pour la production d'une poudre séchée par dispersion selon l'une des revendications 1 à 15 **caractérisé en ce qu'**une solution de dispersion contenant au moins un principe actif pharmaceutique et un formateur de matrice
a) est dispersée en dessous d'une température de 200/120°C ; et
b) la poudre résultante est ensuite soumise à un séchage final sous vide à une température supérieure à 15°C et inférieure à 60°C.

19. Procédé selon la revendication 18 **caractérisé en ce que** le séchage final est accompli à une pression entre 0,01 et 10 mbar.

20. Procédé selon la revendication 18 ou 19 **caractérisé en ce que** la poudre séchée par dispersion est soumise au séchage final pendant au moins 12 heures.

21. Procédé pour la production d'une poudre séchée par dispersion selon l'une des revendications 1 à 15 **caractérisé en ce qu'**une solution de dispersion contenant au moins un principe actif pharmaceutique et un formateur de matrice
a) est dispersée en dessous d'une température de 105/60°C ; et
b) la poudre résultante est ensuite soumise à un séchage final sous vide à une pression d'environ 0,05 à 0,2 mbar, à une température supérieure à 30°C et inférieure à 60°C pendant environ 18 à 30 heures.
